(19)

Europäisches Patentamt
European Patent Office
Office européen des brevets

(11) **EP 4 243 023 A1**

(12) **EUROPEAN PATENT APPLICATION**
published in accordance with Art. 153(4) EPC

(43) Date of publication:
**13.09.2023 Bulletin 2023/37**

(21) Application number: **21889551.4**

(22) Date of filing: **03.11.2021**

(51) International Patent Classification (IPC):
*G16B 15/30* (2019.01)    *G16B 30/10* (2019.01)
*G16B 20/20* (2019.01)    *G16B 40/20* (2019.01)
*C12Q 1/6869* (2018.01)    *G06N 20/00* (2019.01)
*G06N 3/08* (2023.01)

(52) Cooperative Patent Classification (CPC):
**C12Q 1/6869; G06N 3/08; G06N 20/00;
G16B 15/30; G16B 20/20; G16B 30/10;
G16B 40/20**

(86) International application number:
**PCT/KR2021/015800**

(87) International publication number:
**WO 2022/098086 (12.05.2022 Gazette 2022/19)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.11.2020  KR 20200145901**

(71) Applicants:
• **Korea Advanced Institute of Science and Technology**
  **Daejeon 34141 (KR)**

• **GC Genome Corporation**
  **Yongin-si, Gyeonggi-do 16924 (KR)**

(72) Inventors:
• **CHOI, Jung Kyoon**
  **Daejeon 34141 (KR)**
• **KANG, Hyeon Gu**
  **Daejeon 34141 (KR)**
• **CHO, Eun Hae**
  **Yongin-si Gyeonggi-do 16924 (KR)**

(74) Representative: **Mewburn Ellis LLP**
  **Aurora Building**
  **Counterslip**
  **Bristol BS1 6BX (GB)**

(54) **METHOD FOR DETERMINING SENSITIVITY TO PARP INHIBITOR OR DNA DAMAGING AGENT USING NON-FUNCTIONAL TRANSCRIPTOME**

(57)    The present invention relates to a method for determining sensitivity to a PARP inhibitor or a DNA damaging agent by using a non-functional transcriptome, and more particularly, to a method for determining sensitivity to a PARP inhibitor or a DNA damaging agent by extracting nucleic acids from a biological sample to obtain the expression amount of each of non-functional transcriptomes of DNA repair-related genes, and then analyzing the use rate (TU) of each of the non-functional transcriptomes for each gene on the basis of the obtained expression amount. The method for determining sensitivity to a PARP inhibitor or a DNA damaging agent according to the present invention is useful in that sensitivity can be determined in real time with high accuracy because the method uses information of the transcriptomes transcribed in the gene, unlike existing methods which determine sensitivity to a PARP inhibitor or a DNA damaging agent on the basis of genetic variation information.

EP 4 243 023 A1

Fig 1

Obtain RNA fragment (reads) data from biological sample by massive parallel sequencing

↓

Align RNA fragment data to human reference genome

↓

Perform filtering depending on quality of aligned data

↓

Select non−functional transcripts of DNA repair−related genes to be used for determination

↓

Obtain expression levels of non−functional transcripts for each gene

↓

Calculate transcript usage (TU) of non−functional transcripts for each gene

↓

Obtain output value by inputting TU to artificial intelligence model for drug susceptibility determination

↓

Determine drug susceptibility by comparing output value with reference value

**Description**

[Technical Field]

The present invention relates to a method of determining susceptibility to PARP inhibitors or DNA damaging agents using non-functional transcripts, and more particularly to a method of determining susceptibility to a PARP inhibitor or DNA damaging agent by extracting a nucleic acid from a biological sample, obtaining the expression level of each of non-functional transcripts of DNA repair-related genes, and then analyzing the transcript usage (TU) of non-functional transcripts for each gene based on the obtained expression level.

[Background Art]

A biomarker is defined as an indicator able to objectively measure and evaluate susceptibility of drugs to normal biological processes, disease progression, and treatment methods. As research into the relationship between specific genetic mutations and specific diseases has increased with recent development of genetic analysis technology, biomarkers are being redefined as molecular and biological indicators that encompass genes, genetic mutations, and the resulting differences in RNA, protein, and metabolite expression.
Also, in order to classify patients for maximization of therapeutic effects of drugs or minimization of side effects thereof for more effective treatment, a companion diagnostics device (CDx) capable of determining biomarker sensitivity has been developed.
Companion diagnosis is a diagnostic technique for predicting susceptibility of patients to a specific drug therapy in advance. In order to overcome the disadvantages of most existing anticancer drugs that have serious side effects due to action both on cancer cells and normal cells, targeted anticancer drugs that selectively attack a specific target protein have been developed.
However, since targeted anticancer drugs are effective only in cancer patients having a specific target protein even for the same type of cancer, treatment efficiency is very low unless patients having a target molecule are selected.
Also, since targeted anticancer drugs depend on inhibition of cell growth and proliferation rather than cell death, there is a high possibility of developing tolerance due to continuous drug administration over a long period of time. Hence, it is necessary to select patients who are responsive to the drug by analyzing the target of the anticancer drug before drug administration.
Roche, a multinational pharmaceutical company, acquired Genentech, which developed "Herceptin," the first breast cancer-targeted anticancer drug, and "Herceptest," a companion diagnostic kit therefor, and started targeted anticancer drug treatment based on companion diagnosis. The companion diagnostic kit may include a method of confirming overexpression of a specific protein through immunohistochemical testing, such as DAKO, HercepTest, a method of confirming gene amplification of a specific gene through FISH or CISH testing using a DNA probe, such as Ventana Medical Systems, INFORM HER-2/NEU, and a method of determining whether a biomarker gene is mutated using genomic techniques including q-PCR, such as Roche Diagnostics, cobas EGFR mutation test.
Meanwhile, olaparib (AZD2281) is an anticancer drug functioning to suppress abnormal proliferation of cancer cells, and is an inhibitor of "PARP protein". PARP is a protein that repairs damage to DNA in cells, and plays a major role in helping cells complete DNA repair and continue to proliferate. Olaparib inhibits proliferation of cancer cells by suppressing the function of PARP. Olaparib is well known as a targeted therapeutic agent for ovarian cancer and breast cancer, and is particularly known as an effective anticancer drug for cancer patients carrying BRCA1 and BRCA2 genetic mutations.
Specifically, since the effect of anticancer drugs is greatly affected by DNA repair capacity and also since anticancer drugs differ from person to person in view of tolerance and toxicity, selection using appropriate chemosensitivity markers may lead to breakthroughs in anticancer drug therapy. Research into chemosensitivity of individual anticancer drugs adapted for specific genes has recently been actively carried out. However, due to complex actions of biological response-associated factors to specific drugs, diversity of therapeutic agents and administration methods, and difficulty in attaining many samples, there are no remarkable achievements yet.
Myriad genetics has released a product that diagnoses germline BRCA1 and BRCA2 mutations for companion diagnosis of PARP inhibitors (olaparib, talazoparib, and rucaparib). However, this product determines the presence or absence of mutations regardless of the BRCA1/2 gene allele, and the overall response rate (ORR) to PARP inhibitors is only 34%, indicating that companion diagnosis for PARP inhibitors cannot be made sufficiently only by simple germline mutation detection of BRCA1/2.
Foundation Medicine's FoundationFocusCDxBRCA is also a companion diagnostic product that diagnoses the association between BRCA1 and BRCA2 mutations and rucaparib serving as a PARP inhibitor, but the overall response rate (ORR) is only 53.8%, which is still considered to be low.
As described above, HRD-related drug susceptibility may only be partially predicted by BRCA1/2 gene mutations. Accordingly, attempts have been made to predict drug susceptibility by detecting the result caused by HRD rather than the cause of HRD. HRD leaves various scars due to failure to restore damage to the genome. In particular, genomic scar and signature 3 are well-known HRD markers (Gulhan, D. C. et al., Nat. Genet. Vol. 51, pp. 912-919, 2019). Mutational signatures are used to associate genomic patterns of single-nucleotide mutations with specific background factors (Alexandrov, L. B. et al., Nature. Vol. 500, pp. 415-421, 2013). In this sense, signature 3 matches well with HRD. Genomic scars are detected as three chromosomal abnormalities, for example, telomeric allelic imbalances (NtAI), large-scale state transition (LST), and loss of heterozygosity (LOH) (Abkevich, V. et al., Br. J. Cancer. Vol. 107, pp. 1776-1782, 2012; Popova, T. et al., Cancer Res. Vol. 72, pp. 5454-5462, 2012 (Nicolai J. Birkbak. CANCER Discov. Vol. 2, 367, 2012).

Analysis of genomic HRD (gHRD) is inevitably affected by functional recovery of HR. Specifically, occurrence of another mutation that restores BRCA1/2 function is responsible for approximately half of platinum drug-resistant cases in ovarian cancer (Norquist, B. et al., J. Clin. Oncol. Vol. 29, pp. 3008-3015, 2011). Also, a BRCA1/2 independent mechanism regarding PARP inhibitor resistance has been known (Chaudhuri, A. R. et al., Nature. Vol. 535, pp. 382-387, 2016). As such, there is a problem in that the genomic scar that first occurred is still detected even in the situation where the HR function is restored. Moreover, it is known that false positives further increase due to technical problems in calculating mutational signatures (Maura, F. et al., Nat. Commun. Vol. 10, 2019).

Owing to the above problems, most patients expected to respond to DNA damaging agents or PARP inhibitors do not actually respond (Watkins, J. A. et al., Breast Cancer Research. Vol. 16, pp. 1-11, 2014).

Therefore, there is a need for new technology capable of monitoring the patient's HRD status in real time, such as methods of removing drug-resistant cases from gHRD prediction results using biomarkers that measure the functional status of HRD.

Accordingly, the present inventors have made great efforts to develop a method of determining susceptibility to PARP inhibitors or DNA damaging agents including platinum with high accuracy, and ascertained that, when non-functional transcripts that are estimated to be translated into proteins which are not normally translated or function of which is lost for each gene are extracted using transcript structure information of DNA repair-related genes and then the expression levels thereof are analyzed, susceptibility to PARP inhibitors or DNA damaging agents may be determined with lower false positives and higher accuracy than methods of detecting HRD at the genetic mutation level, thus culminating in the present invention.

[Disclosure]

It is an object of the present invention to provide a method of determining susceptibility to a PARP inhibitor and DNA damaging agent using non-functional transcripts.

It is another object of the present invention to provide an apparatus for determining susceptibility to a PARP inhibitor and DNA damaging agent using non-functional transcripts.

It is still another object of the present invention to provide a computer-readable storage medium including instructions configured to be executed by a processor for determining susceptibility to a PARP inhibitor and DNA damaging agent by the method described above.

It is yet another object of the present invention to provide a targeted RNA sequencing kit using the method described above.

In order to accomplish the above objects, the present invention provides a method of determining susceptibility to a PARP inhibitor or DNA damaging agent including a) extracting a nucleic acid from a biological sample and then obtaining the expression level of each of non-functional transcripts of DNA repair-related genes, b) calculating transcript usage (TU) of non-functional transcripts for each gene based on the obtained expression level, and c) determining that there is susceptibility to a PARP (poly ADP-ribose polymerase) inhibitor or DNA damaging agent (genotoxic drug) when a value obtained by analyzing the calculated TU is greater than or equal to a reference value.

In addition, the present invention provides an apparatus for determining susceptibility to a PARP inhibitor or DNA damaging agent for use in the method of determining susceptibility to a PARP inhibitor or DNA damaging agent, and a computer-readable recording medium including instructions for performing the method described above.

In addition, the present invention provides a targeted RNA sequencing (targeted RNA-Seq) kit for use in the method of determining susceptibility to a PARP inhibitor or DNA damaging agent.

In addition, the present invention provides a method of treating cancer including administering a PARP inhibitor or DNA damaging agent to a patient determined to have susceptibility based on the method of determining susceptibility to a PARP inhibitor or DNA damaging agent.

[Description of Drawings]

FIG. 1 shows an overall flowchart for determining susceptibility to a PARP inhibitor or DNA damaging agent according to the present invention;

FIG. 2 shows a conceptual process for calculating transcript usage (TU) according to the present invention;

FIG. 3 shows results in which minor isoforms overexpressed in gHRD(+) samples lead to a statistically significant loss of protein function according to an embodiment of the present invention;

FIG. 4 shows results in which genes in which a specific minor isoform is overexpressed in gHRD(+) samples are statistically significantly associated with DNA repair function according to an embodiment of the present invention;

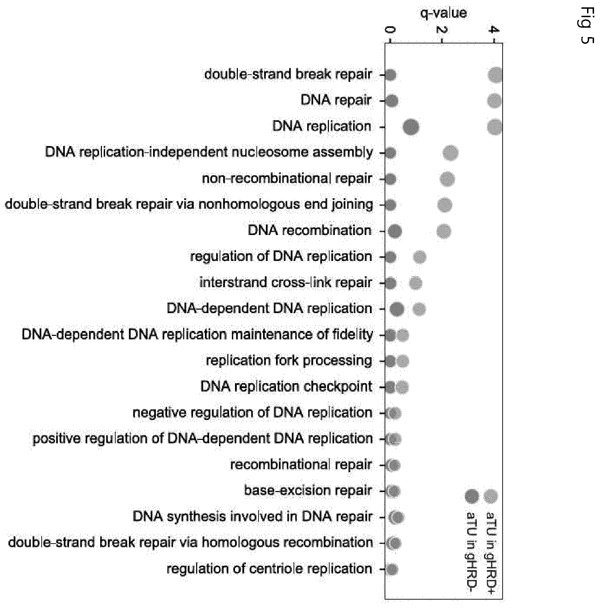

FIG. 5 shows results in which genes in which minor isoforms are overexpressed in gHRD(+) samples are statistically significantly associated with various sub-functions of DNA repair according to an embodiment of the present invention;

FIG. 6 shows a comparison of results predicted by gHRD for drug susceptibility of breast cancer cell lines in an embodiment of the present invention with results predicted by tHRD obtained based on 104 transcripts of 36 genes related to breast cancer, drug susceptibility between gHRD- and tHRD-positive and negative cell lines being represented as IC50;

FIG. 7 shows a comparison in view of precision and recall of results predicted by gHRD for drug susceptibility of breast cancer cell lines in an embodiment of the present invention and results predicted by tHRD obtained based on 104 transcripts of 36 genes related to breast cancer, the blue dotted line and solid line representing performance before and after gHRD (signature 3) correction, respectively, and the red line representing tHRD performance;

FIG. 8 shows a comparison of results of calculating tHRD with 20 major transcripts among 104 transcripts related to breast cancer discovered in an embodiment of the present invention with results of gHRD drug response prediction;

FIG. 9 shows a comparison of results of calculating tHRD with 10 major transcripts among 104 transcripts related to breast cancer discovered in an embodiment of the present invention with results of gHRD drug response prediction;

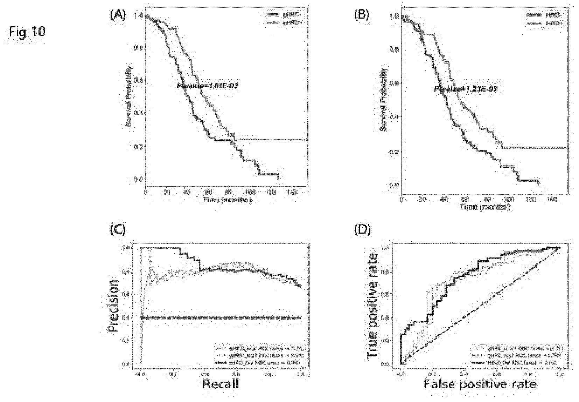

FIGs. 10 (A) and (C) show results of predicting platinum response in ovarian cancer patients by gHRD, and (B) and (D) show results of predicting platinum response in ovarian cancer patients by tHRD based on 89 transcripts of 25 genes related to ovarian cancer discovered in an embodiment of the present invention, in which (A) and (B) show results of comparing the survival rates of gHRD- and tHRD-positive and negative patients, and (C) and (D) show results of analyzing the precision and recall and the receiver operating characteristics in the two methods, the blue dotted line representing performance of gHRD (scar), the blue solid line representing performance of gHRD (signature 3), and the red line representing tHRD performance;

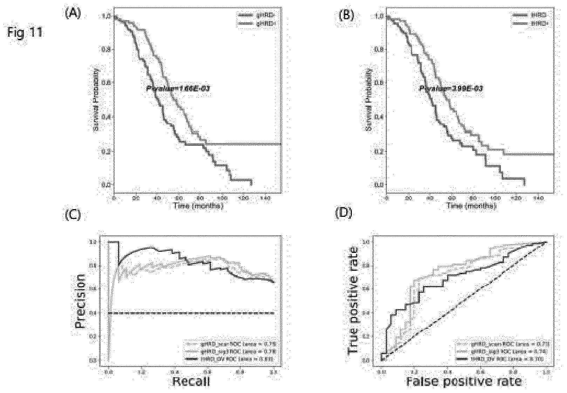

FIGs. 11 (A) and (C) show results of predicting platinum response in ovarian cancer patients by gHRD, and (B) and (D) show results of predicting platinum response in ovarian cancer patients by tHRD based on 10 major transcripts among 89 transcripts of 25 genes related to ovarian cancer discovered in an embodiment of the present invention, in which (A) and (B) show results of comparing the survival rates of gHRD- and tHRD-positive and negative patients, and (C) and (D) show results of analyzing the precision and recall and the receiver operating characteristics in the two methods, the blue dotted line representing performance of gHRD (scar), the blue solid line representing performance of gHRD (signature 3), and the red line representing tHRD performance;

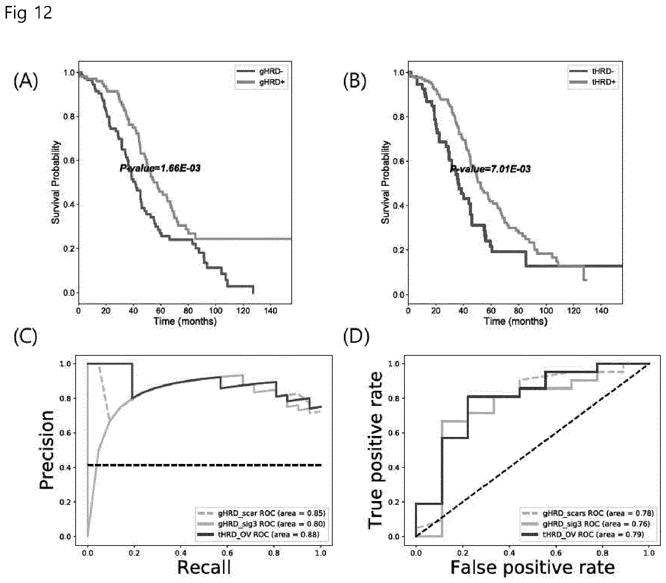

FIG. 12 shows results of comparing performance of tHRD models learned based on platinum response rather than gHRD with performance predicted by gHRD for platinum response, (A) and (B) showing results of comparing survival rates after platinum treatment of patients classified as positive and negative in the learned gHRD and tHRD models, and (C) and (D) showing results of analyzing the precision and recall and the receiver operating characteristics in the two methods, the blue dotted line representing performance of gHRD (scar), the blue solid line representing performance of gHRD (signature 3), and the red line representing tHRD performance; and

FIG. 13 shows results of survival analysis using the cancer metastasis period after platinum drug treatment by classifying actual ovarian cancer patients with the tHRD models constructed in an embodiment of the present invention, (A) representing 12 months, (B) representing 24 months, and (C) representing the entire period.

[Mode for Invention]

Unless otherwise defined, all technical and scientific terms used herein have the same meanings as typically understood by those skilled in the art to which the present invention belongs. In general, the nomenclature used herein and experimental methods described below are well known in the art and are typical.

Terms such as first, second, A, B, and the like may be used to describe various components, but the components are not limited by the above terms, and these terms are used only for the purpose of distinguishing one component from another component. For example, the first component may be referred to as the second component, and similarly, the second component may also be referred to as the first component, without departing from the scope of the technology to be described below. The term "and/or" includes a combination of a plurality of related listed items or any item of a plurality of related listed items.

For the terms used herein, the singular forms are intended to include the plural forms as well, unless the context clearly indicates otherwise, and the terms such as "comprise", "include", and the like specify the presence of stated features, numbers, steps, operations, elements, components, or combinations thereof, but do not preclude the presence or addition of one or more other features, numbers, steps, operations, elements, components, or combinations thereof.

Before a detailed description of the drawings, it is intended to clarify that classification of the constituents in the present specification is merely a division according to the main function that each constituent is responsible for. Specifically, two or more constituents to be described below may be combined into one constituent, or one constituent may be divided into two or more according to the more subdivided function. In addition, each of the constituents to be described below may additionally perform some or all of the functions of other constituents in addition to the main function it is responsible for, and some of the main functions of individual constituents may be dedicated to and performed by other constituents.

In addition, in performing a method or operation, individual steps constituting the method may occur in a different order from the specified order unless a specific order is clearly described in context. Specifically, individual steps may occur in the same order as specified, may be performed substantially simultaneously, or may be performed in reverse order.

The present invention is intended to confirm that a method of determining susceptibility to a PARP inhibitor or DNA damaging agent, including calculating transcript usage (TU) of each non-functional transcript based on the expression levels of non-functional transcripts of genes obtained from samples and then comparing a value obtained by analyzing the calculated TU with a reference value, is capable of determining the susceptibility to a PARP inhibitor or DNA damaging agent with high accuracy compared to methods of predicting susceptibility with genomic scars.

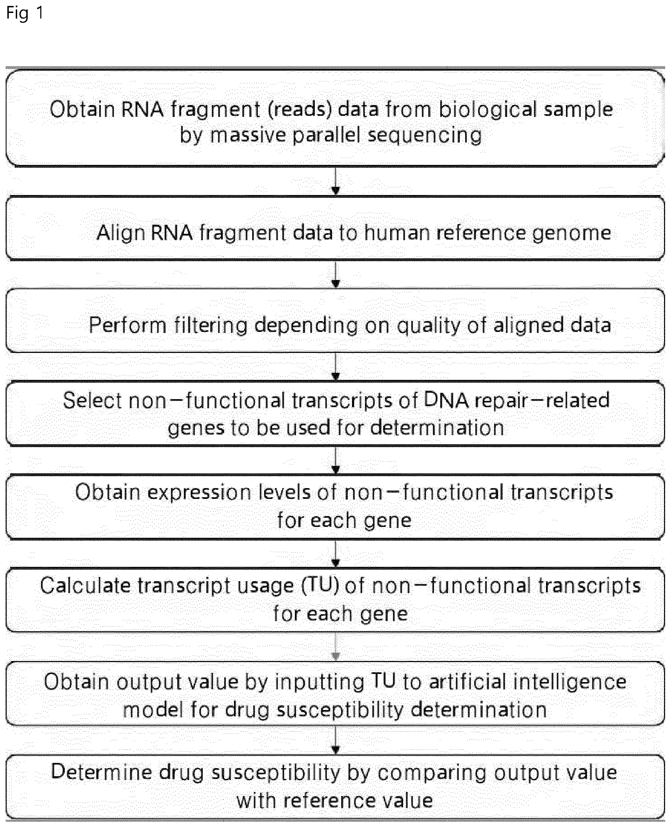

In an embodiment of the present invention, genomic HRD was obtained from breast cancer/ovarian cancer patient data and then classified into gHRD(+) and gHRD(-) groups, major and minor isoforms of genes were distinguished based on a transcript structure database, the transcript usage (TU) of transcripts for each minor isoform was calculated, and genes showing aberrant TU (aTU) in which the minor isoform is overexpressed in gHRD(+) were discovered and determined to be mainly DNA repair-related genes. Then, genes to be used for optimizing drug susceptibility determination for each cancer type and minor isoforms thereof were selected, and the TU values of these minor isoforms were used as input data for a random forest model to thus train an artificial intelligence model that determines the presence or absence of HRD, whereby a method of determining that susceptibility to a PARP inhibitor or DNA damaging agent is high when tHRD is positive was developed (FIG. 1).

Accordingly, an aspect of the present invention pertains to a method of determining susceptibility to a PARP inhibitor or DNA damaging agent, including:

> a) extracting a nucleic acid from a biological sample and then obtaining the expression level of each of non-functional transcripts of DNA repair-related genes;
> b) calculating transcript usage (TU) of non-functional transcripts for each gene based on the obtained expression level; and
> c) determining that there is susceptibility to a PARP (poly ADP-ribose polymerase) inhibitor or DNA damaging agent (genotoxic drug) when a value obtained by analyzing the calculated TU is greater than or equal to a reference value.

In the present invention, the nucleic acid may be RNA, but is not limited thereto.

In the present invention, step a) may be performed through a method including:

> a-i) collecting nucleic acid from blood, semen, vaginal cells, hair, saliva, urine, oral cells, cancer tissue cells, FFPE samples, and mixtures thereof;
> a-ii) obtaining purified nucleic acid by removing proteins, fats, and other residues from the collected nucleic acid using a salting-out method, a column chromatography method, or a bead method;
> a-iii) constructing a library by enriching DNA repair-related genes for the purified nucleic acid;
> a-iv) reacting the constructed library in a next-generation sequencer; and
> a-v) acquiring nucleic acid sequence information (reads) from the next-generation sequencer.

In the present invention, any method for enriching the DNA repair-related genes may be used so long as it is a technique known to those skilled in the art, and includes, but is not limited to, a probe-based capture method, a primer-based amplification method, etc.

In the present invention, the library may be composed of cDNA, but the present invention is not limited thereto.

In the present invention, the next-generation sequencer may be used for any sequencing method known in the art. Sequencing of nucleic acids isolated by the selection method is typically performed using next-generation sequencing (NGS). Next-generation sequencing includes any sequencing method that determines the nucleotide sequence of either individual nucleic acid molecules or clonally expanded proxies for individual nucleic acid molecules in a high throughput fashion (e.g. $10^5$ or more molecules are sequenced simultaneously). In one embodiment, the relative abundance of nucleic acid species in the library may be estimated by counting the relative number of occurrences of the cognate sequences thereof in data generated by sequencing experiments. Next-generation sequencing methods are known in the art and are described, for example, in Metzker, M. (2010) Nature Biotechnology Reviews 11:31-46, which is incorporated herein by reference.

In one embodiment, next-generation sequencing is performed to determine the nucleotide sequences of individual nucleic acid molecules (e.g. HeliScope Gene Sequencing system from Helicos BioSciences and PacBio RS system from Pacific Biosciences). In other embodiments, sequencing, for example, massive parallel short-read sequencing that yields more bases of sequence per sequencing unit (e.g. Solexa sequencer from Illumina Inc., San Diego, California) than other sequencing methods yielding fewer but longer reads determines the nucleotide sequence of clonally expanded proxies for individual nucleic acid molecules (e.g. Solexa sequencer from Illumina Inc., San Diego, California; 454 Life Sciences (Branford, Connecticut) and Ion Torrent) . Other methods or machines for next-generation sequencing include, but are not limited to, sequencers provided by 454 Life Sciences (Branford, Connecticut), Applied Biosystems (Foster City, California; SOLiD Sequencer), Helicos BioSciences Corporation (Cambridge, Massachusetts), and emulsion and microfluidic sequencing technology nanodroplets (e.g. GnuBio Droplets).

Platforms for next-generation sequencing include, but are not limited to, Genome Sequencer (GS) FLX system from Roche/454, Genome Analyzer (GA) from Illumina/Solexa, Support Oligonucleotide Ligation Detection (SOLiD) system from Life/APG, G.007 system from Polonator, HeliScope Gene Sequencing system from Helicos BioSciences, and PacBio RS system from Pacific Biosciences.

In the present invention, the terms "sample", "tissue sample", "patient sample", "patient cell or tissue sample", "cancer tissue cell", "FFPE sample", or "specimen" refer to a collection of similar cells obtained from tissue or circulating cells of subjects or patients. The source of the tissue sample may be solid tissue from fresh, frozen, and/or preserved organs, tissue samples, biopsies or aspirations; blood or any blood component; bodily fluids such as cerebrospinal fluid, amniotic fluid, peritoneal fluid, or interstitial fluid; or cells from any point during subject's pregnancy or development. The tissue sample may contain compounds that are not naturally intermixed with tissue in nature, such as preservatives, anticoagulants, buffers, fixatives, nutrients, antibiotics, and the like. In one embodiment, the sample is prepared as a frozen sample or as a formaldehyde- or paraformaldehyde-fixed paraffin-embedded (FFPE) tissue. For example, the sample may be embedded in a matrix, such as a FFPE block or a frozen sample.

In one embodiment, the sample is a tumor sample and includes, for example, one or more precancerous or malignant cells. In a certain embodiment, the sample, for example, a tumor sample, is obtained from a solid tumor, soft tissue tumor, or metastatic lesion. In another embodiment, the sample, for example, a tumor sample, includes tissue or cells from surgical resection. In still another embodiment, the sample, for example, a tumor sample, includes one or more circulating tumor cells (CTCs) (e.g. CTCs obtained from a blood sample).

In the present invention, obtaining the expression levels of the non-functional transcripts for each gene may be performed without limitation through any NGS-based RNA-seq data analysis method known to those skilled in the art.

In the present invention, the DNA repair-related genes may be used without limitation so long as they are genes known to be related to DNA repair, and preferably include, but are not limited to, at least 10 genes selected from the group consisting of ABL1, ALKBH1, APEX1, APTX, ASF1A, ATM, ATP23, ATR, ATRX, ATXN3, BLM, BRCA1, BRCA2, BTG2, CCNO, CDKN2D, CEBPG, CIB1, CSNK1D, CSNK1E, DDB1, DDB2, ERCC1, ERCC2, ERCC3, ERCC4, ERCC5, ERCC6, ERCC8, EXO1, FANCA, FANCC, FANCG, FEN1, GADD45A, GADD45G, GTF2H1, GTF2H4, HMGB1, HMGB1P10, HMGB2, HUS1, IGHMBP2, KAT5, LIG1, LIG3, LIG4, MLH1, MMS19, MNAT1, MPG, MRE11, MSH2, MSH3, MSH5, MSH6, MUTYH, NBN, NHEJ1, NTHL1, OGG1, PARP1, PARP3, PMS1, PMS2, PMS2P1, PNKP, POLA1, POLD1, POLE, POLE2, POLG, POLH, POLI, POLL, POLQ, PRKCG, RAD1, RAD17, RAD21, RAD23A, RAD23B, RAD50, RAD51, RAD51B, RAD51C, RAD52, RAD54B, RAD54L, RAD9A, RBBP8, RECQL, RECQL4, RECQL5, REV1, RFC3, RPA1, RPAIN, RUVBL2, SETX, SMC1A, SMUG1, SOD1, SUMO1, TDG, TNP1, TP53, TP73, TREX2, UBE2A, UBE2B, UBE2N, UBE2V1, UBE2V2, UNG, UPF1, UVRAG, VCP, WRNIP1, XAB2, XPC, XRCC2, XRCC3, XRCC4, XRCC6, BABAM2, BRIP1, CDCA5, CHEK1, DCLRE1C, FANCB, FANCI, MGME1, MND1, MUS81, NEIL1, PARP9, RAD51AP1, RFC4, SMARCB1, TICRR, TRIP13, UBE2T, USP47, ABRAXAS1, ASCC1, CHEK2, NSMCE4A, PARP2, RAD51AP1, RHNO1, RMI2, RPS3, TNKS1BP1, UBB, UIMC1, and USP45.

In the present invention, when the PARP inhibitor or DNA damaging agent is applied to breast cancer, the DNA repair-related genes may include, but are not limited to, at least 10 genes selected from the group consisting of ALKBH2, ATXN3, BABAM2, BRIP1, CDCA5, CHEK1, DCLRE1C, DDB2, ERCC1, EXO1, FANCB, FANCC, FANCI, FEN1, KAT5, MGME1, MND1, MSH5, MUS81, NEIL1, PARP3, PARP9, POLD1, RAD51, RAD51AP1, RAD54L, RFC4, RPAIN, SMARCB1, SMC1A, TICRR, TRIP13, UBE2T, UBE2V2, and USP47.

In the present invention, when the PARP inhibitor or DNA damaging agent is applied to ovarian cancer, the DNA repair-related genes may include, but are not limited to, at least 10 genes selected from the group consisting of ABRAXAS1, ASCC1, BLM, CHEK2, ERCC1, EXO1, GADD45A, MUTYH, NSMCE4A, PARP2, POLE2, RAD51AP1, RAD51B, RECQL4, RHNO1, RMI2, RPS3, SUMO1, TNKS1BP1, UBB, UBE2A, UIMC1, USP45, VCP, and XPC.

In the present invention, the non-functional transcripts in step a) may be minor isoforms, and the minor isoforms may include, but are not limited to, transcripts that are estimated to be translated into proteins which are not normally translated or functions of which are lost, or transcripts that are not used for protein translation.

In the present invention, the non-functional transcripts in step a) may be transcripts remaining after removing transcripts (e.g. transcripts corresponding to principals (1 to 5) in APRIS database) encoding well-conserved proteins for each gene in a known database (e.g. APRIS database).

For the non-functional transcripts of the present invention, selecting non-functional transcripts derived from genes having an average count per million (CPM) value of 1 or more in order to increase the accuracy of subsequent analysis may be further performed.

In the present invention, in calculating the transcript usage (TU) of non-functional transcripts for each gene in step b), the TU value is a ratio in which the sum of TPM values of all transcripts occurring in one gene is used as the denominator and the TPM of each transcript is used as the numerator, and is preferably calculated using Equation 1 below:

$$\text{Equation 1:} \qquad \mathbf{TU}_t = \mathbf{TPM}_t / \sum_t \mathbf{TPM}_t$$

Here, TPM represents transcripts per million.

In the present invention, after obtaining the expression levels of non-functional transcripts for each gene, in order to increase the accuracy of subsequent analysis, selecting specific transcripts having a TU value of 1 to 5% or more, preferably 3% or more, among the selected genes may be further included.

In the present invention, obtaining the value by analyzing the calculated TU in step c) may be performed in a manner in which TU values of non-functional transcripts overexpressed in a sample known to be genomic homologous recombination deficiency (gHRD) positive or drug responsive are multiplied by a specific weight and summed to obtain a determination value in a specific range, or may be performed using a decision-making process that makes a final decision according to an aspect that the TU value of each of non-functional transcripts exceeds a specific reference value.

In the present invention, the specific weight may be used without limitation, so long as it is a value for obtaining a determination value in a specific range, and may be increased with an increase in the TU values of non-functional transcripts overexpressed in a sample known to be gHRD positive or drug responsive.

In the present invention, the determination value in the specific range may be used without limitation, so long as it is able to determine positive or negative susceptibility to a PARP inhibitor or DNA damaging agent, and may be a value that is preferably normalized between 0 and 1, but is not limited thereto.

In the present invention, the specific reference value may be determined based on the TU values of the non-functional transcripts obtained from a sample known to be gHRD positive or drug responsive, preferably overexpressed non-functional transcripts, but the present invention is not limited thereto.

For example, in the present invention, obtaining the value by analyzing the calculated TU in step c) may be performed in a manner in which the TU values of non-functional transcripts corresponding to the non-functional transcripts overexpressed in the sample known to be gHRD positive or drug responsive are multiplied by a weight, summed, and normalized to a value between 0 and 1.

In the present invention, the weight may be used without limitation, so long as it is a value assigned so that the summed value is between 0 and 1, and may be increased with an increase in the TU values of non-functional transcripts overexpressed in a sample known to be gHRD positive or drug responsive.

For example, in the present invention, obtaining the value by analyzing the calculated TU in step c) may be performed using a decision-making process in which the TU value of each of non-functional transcripts is determined based on TU values of non-functional transcripts obtained from a sample known to be gHRD positive or drug responsive.

In the present invention, obtaining the value by analyzing the calculated TU in step c) may be performed using an artificial intelligence model.

In the present invention, the artificial intelligence model is capable of constructing a prediction model for the relationship between gHRD or drug response and the expression levels of non-functional transcripts for each gene through machine learning using the weights for the TU values of non-functional transcripts for each gene obtained from a sample already known to be gHRD positive or drug responsive and the reference value for the determination value obtained therefrom, or the reference value for each TU value for decision making and the decision-making process structure.

Specifically, the artificial intelligence model of the present invention is capable of constructing a prediction model through machine learning using combinations of weights that may be assigned to the TU values of non-functional transcripts for each gene overexpressed in a gHRD-positive or drug-responsive sample and the reference value for the determination value resulting from summing the weighted TU values so as to reflect transcript expression patterns of various patients, or is capable of constructing a prediction model through machine learning by structuring the decision-making process of comparing the TU values of non-functional transcripts for each gene overexpressed in a gHRD-positive or drug-responsive sample with a reference value therefor so as to reflect transcript expression patterns of various patients.

For example, the artificial intelligence model of the present invention is capable of constructing a prediction model through learning by multiplying the TU values of non-functional transcripts for each gene overexpressed in a gHRD-positive or drug-responsive sample by the weight so that the summed value is close to 1.

In the present invention, the transcript expression patterns of various patients may mean the types and patterns of transcripts that are overexpressed depending on the patients.

Therefore, when a combination of specific TU values is entered as input data, a decision-making value or determination value close to positive is output in combinations similar to the gHRD-positive or drug-responsive sample, and thus, when the output value is greater than or equal to the reference value, it is determined that there is susceptibility to a PARP inhibitor or DNA damaging agent.

In the present invention, the reference value may be used without limitation, so long as it is able to determine the susceptibility of the sample to a PARP inhibitor or DNA damaging agent, and is preferably 0.5 to 1, more preferably 0.5 to 0.8, most preferably 0.5, but is not limited thereto.

In the present invention, the machine learning may be used without limitation so long as it is supervised learning, and is preferably performed through at least one process selected from the group consisting of K-nearest neighbors, linear regression, logistic regression, support vector machine (SVM), decision tree, random forest, and neural network, but the present invention is not limited thereto.

In the present invention, when the machine learning is performed through random forest, the sub-decision tree is learned so as to minimize Gini impurity calculated using Equation 2 below:

Equation 2:

$$G_i = 1 - \sum_{k=1}^{n} p_{i,k}{}^2$$

Gini impurity

Here, i represents the ith node, n represents the number of output classes, k represents the kth class of output values, and $p_{i,k}$ represents the ratio of samples belonging to class k among the training samples at the ith node.

In the present invention, the random forest creates multiple training data that allow overlap based on one training data set, trains multiple sub-decision trees based thereon, and then calculates the average of these model predictions to obtain a final prediction value.

In the present invention, the machine learning based on gHRD is performed to predict an HRD status using the TU values of minor isoforms of DNA repair-related genes as input data. Here, the HRD status is classified based on gHRD.

The gHRD method is known to have many false positives that show positive even when HR function is already restored due to additional mutations of BRCA1/2 or that show positive regardless of HR function due to technical artifacts. In fact, it has been reported that the susceptibility of samples predicted to be gHRD(+) to PARP inhibitors or platinum drugs is less than 500. Therefore, learning is possible to determine that only samples having transcripts with statistically significantly high TUs of minor isoforms have functional HRD even within the gHRD(+) group and are actually susceptible to PARP inhibitors or DNA damaging agents. In addition, as the reference value of gHRD increases due to low precision, the recall decreases due to occurrence of false negatives that do not pass the reference value even in the presence of functional HRD and drug susceptibility. Therefore, it is possible to improve both precision and recall by discriminating only cases that actually show functional aTU pattern, ultimately increasing overall accuracy.

In the present invention, the machine learning based on drug response is performed to predict response to PARP inhibitors or DNA damaging agents such as platinum using the TU values of minor isoforms of DNA repair-related genes as input data. Here, drug response is based on cancer progression within 6 months after drug treatment in a typical manner. Accordingly, patients whose cancer progressed within 6 months are classified as resistant patients, and patients who did not progress to cancer are classified as responsive patients.

In the present invention, samples judged to be HRD positive or drug responsive by the artificial intelligence model are defined as having "transcriptional homologous recombination deficiency (tHRD)".

In the present invention, a difference in TU between the group showing HRD (gHRD(+)) and the group not showing HRD (gHRD(-)) at the genome level based on "genomic scar" and "signature 3" was statistically analyzed, and thus, relative overexpression of a specific minor isoform is defined as aberrant TU (aTU), overexpression thereof in the gHRD(+) group is defined as "aTU in gHRD(+)", and overexpression thereof in the gHRD(-) group is defined as "aTU in gHRD(-)".

Theoretically, both an isoform corresponding to aTU in gHRD(+) and an isoform corresponding to aTU in gHRD(-) may appear in the same gene, but in practice, it can be found that aTU in gHRD(+) appears much more frequently in DNA repair-related genes.

In the present invention, the terms "genomic scar" and "signature 3" refer to chromosomal rearrangement or mutation that appears in cells in which the function of homologous recombination has been lost, and mean chromosomal rearrangement or mutation occurring at the whole genome level regardless of which part of the homologous recombination pathway has been lost, and may be confirmed by array-based comparative genomic hybridization (aCGH), single-nucleotide polymorphism (SNP) genotyping, and next-generation sequencing (NGS).

In the present invention, the PARP inhibitor may be used without limitation, so long as it is able to inhibit the activity of PARP protein, but is preferably a natural compound, synthetic compound, DNA, RNA, peptide, enzyme, ligand, cell extract, or secretion of a mammal, which inhibits the activity of PARP protein, and is more preferably selected from the group consisting of AZD2281 (olaparib), ABT888 (veliparib), AG014699 (rucaparib), MK-4827 (niraparib), BMN-673 (talazoparib), BSI201 (iniparib), BGP15 (O-(3-piperidino-2-hydroxy-1-propyl)nicotinic amidoxime), INO1001 (3-aminobenzamide), ONO2231, nicotinamide, 3-aminobenzamide, 3,4-dihydro-5-[4-(1-piperidinyl)butoxy]-1(2H)-isoquinolone, benzamide, quinolone, isoquinolone, benzopyrone, cyclic benzamide, benzimidazole, indole, and phenanthridinone, but is not limited thereto.

In the present invention, the cancer disease targeted by the PARP inhibitor may be selected from the group consisting of ACTH-producing tumor, acute lymphocytic or lymphoblastic leukemia, acute or chronic lymphocytic leukemia, acute non-lymphocytic leukemia, bladder cancer, brain tumor, breast cancer, cancer of cervix, chronic myelogenous leukemia, lymphoma, endometriosis, esophageal cancer, Ewing's sarcoma, tongue cancer, Hopkins lymphoma, Kaposis' sarcoma, kidney cancer, liver cancer, lung cancer, mesothelioma, multiple myeloma, neuroblastoma, non-Hopkin's lymphoma, osteosarcoma, ovarian cancer, mammary cancer, prostate cancer, pancreatic cancer, colorectal cancer, penile cancer, retinoblastoma, skin cancer, stomach cancer, thyroid cancer, uterine cancer, testicular cancer, Wilms' tumor, and trophoblastoma, but is limited thereto.

In the present invention, the DNA damaging agent may be used without limitation, so long as it is a drug that causes DNA modification, such as inducing crosslinking, double-strand break, intercalation, etc. of DNA in cells, and is preferably selected from the group consisting of bleomycin, cisplatin, carboplatin, oxaliplatin, nedaplatin, doxorubicin, etoposide, and SN38, but is limited thereto.

The method of determining susceptibility to a PARP inhibitor or DNA damaging agent according to the present invention may include the following steps according to an embodiment, but the present invention is not limited thereto (FIG. 1):

(1) obtaining RNA fragment (reads) data from a biological sample by massive parallel sequencing;
(2) aligning the RNA fragment data to a human reference genome;
(3) performing filtering depending on quality of the aligned data;
(4) selecting non-functional transcripts of DNA repair-related genes to be used for determination;
(5) obtaining the expression levels of non-functional transcripts for each gene;
(6) calculating transcript usage (TU) of non-functional transcripts for each gene;
(7) obtaining an output value by inputting the TU to an artificial intelligence model for drug susceptibility determination; and
(8) determining drug susceptibility by comparing the output value with a reference value

Another aspect of the present invention pertains to an apparatus for determining susceptibility to a PARP inhibitor or DNA damaging agent for use in the method of determining susceptibility to a PARP inhibitor or DNA damaging agent according to the present invention, the apparatus including:

(1) an information acquisition unit configured to extract a nucleic acid from a biological sample and obtain the expression level of each of non-functional transcripts of DNA repair-related genes;
(2) a calculation unit configured to calculate transcript usage (TU) of non-functional transcripts for each gene based on the obtained expression level; and
(3) a susceptibility determination unit configured to determine that there is susceptibility to a PARP (poly ADP-ribose polymerase) inhibitor or DNA damaging agent (genotoxic drug) when a value obtained by analyzing the calculated TU is greater than or equal to a reference value.

In the present invention, the information acquisition unit may include a data receiving unit configured to receive RNA fragment (reads) data obtained from an independently produced biological sample through massive parallel sequencing, a data alignment unit configured to align the received data to a reference genome, a filtering unit configured to perform filtering depending on data quality, and an expression level acquisition unit configured to select non-functional transcripts of DNA repair-related genes and acquire expression levels thereof, but the present invention is not limited thereto.

Still another aspect of the present invention pertains to a computer-readable recording medium for use in the method of determining susceptibility to a PARP inhibitor or DNA damaging agent according to the present invention, in which the medium includes instructions configured to be executed by a processor for determining susceptibility to a PARP inhibitor or DNA damaging agent, including:

a) extracting a nucleic acid from a biological sample and then obtaining the expression level of each of non-functional transcripts of DNA repair-related genes;

b) calculating transcript usage (TU) of non-functional transcripts for each gene based on the obtained expression level; and

c) determining that there is susceptibility to a PARP (poly ADP-ribose polymerase) inhibitor or DNA damaging agent (genotoxic drug) when a value obtained by analyzing the calculated TU is greater than or equal to a reference value.

Yet another aspect of the present invention pertains to a computer for use in performing the method according to the present invention. In one embodiment, the computer includes at least one processor coupled to a chipset. Also, a memory, storage device, keyboard, graphics adapter, pointing device, and network adapter are connected to the chipset. In one embodiment, performance of the chipset is enabled by a memory controller hub and an I/O controller hub. In another embodiment, the memory may be used by being directly connected to the processor instead of the chipset. The storage device is any device capable of holding data, including a hard drive, CD-ROM (compact disk read-only memory), DVD, or other memory devices. The memory handles data and instructions used by the processor. The pointing device may be a mouse, track ball, or other type of pointing device, and is used in combination with a keyboard to transmit the input data to the computer system. The graphics adapter presents images and other information on the display. The network adapter is connected to the computer system through a local-area or long-distance network. The computer used herein is not limited to the above configuration, may not include some components or may include additional components, and may also be a part of a storage area network (SAN), and the computer of the present application may be configured to be suitable for execution of a module in a program for performing the method according to the present invention.

As used herein, the module may mean a functional or structural combination of hardware for performing the technical idea according to the present application and software for driving the hardware. For example, those skilled in the art may easily infer that the module may mean a predetermined code and a logical unit of hardware resources for executing the predetermined code, and does not necessarily mean a physically connected code or a single type of hardware.

The method according to the present disclosure may be implemented in hardware, firmware, software, or a combination thereof. When implemented in software, the storage medium includes any storage or transmission medium in a form readable by a device such as a computer. Examples of a computer-readable medium may include ROM (read only memory), RAM (random access memory), magnetic disk storage media, optical storage media, flash memory devices, and other electrical, optical, or acoustic signal transmission media.

In this aspect, the present application also pertains to a computer-readable medium including an execution module configured to execute a processor to perform an operation including the steps according to the present disclosure described above.

Still yet another aspect of the present invention pertains to a targeted RNA sequencing (targeted RNA-Seq) kit for use in the method of determining susceptibility to a PARP inhibitor or DNA damaging agent in the present invention, the kit including:

probes configured to capture transcripts of DNA repair-related genes and primers configured to amplify the captured transcripts.

In the present invention, the transcripts may be non-functional transcripts, but are not limited thereto.

In the present invention, the kit may selectively include a buffer, DNA polymerase, DNA polymerase cofactor, and a reagent necessary for carrying out nucleic acid amplification reaction (e.g. polymerase chain reaction) such as deoxyribonucleotide-5-triphosphate (dNTP). Also, the kit of the present invention may selectively include various oligonucleotide molecules, reverse transcriptase, various buffers and reagents, and antibodies that inhibit DNA polymerase activity. Also, the optimum amount of the reagent used in a certain reaction of the kit may be easily determined by those skilled in the art having learned the disclosure herein. Typically, the apparatus of the present invention may be manufactured in a separate package or compartment including the aforementioned components.

In one embodiment, the kit may include a compartmental carrier member for holding a sample, a container containing a reagent, a container containing a probe or primer, and a container containing a probe for detecting the amplification product.

The carrier member is suitable for containing one or more containers, such as bottles and tubes, and individual containers contain independent components for use in the method of the present invention. In the context of the present invention, the required agent in the container may be readily dispensed by a person of ordinary skill in the art.

The present invention also pertains to a method of treating cancer including administering a PARP inhibitor or DNA damaging agent to a patient determined to have susceptibility based on the method of determining susceptibility to the PARP inhibitor or DNA damaging agent.

Examples

A better understanding of the present invention may be obtained through the following examples. These examples are set forth to illustrate the present invention, and are not to be construed as limiting the scope of the present invention, as is obvious to those skilled in the art.

**Example 1. Acquisition of genomic data and transcript data of breast and ovarian cancer patients**

Data on 645 breast cancer samples for which whole-exome sequencing (WES), genotyping data (Affymetrix SNP chip), and RNA sequencing (RNA-seq) data are available and 315 ovarian cancer samples for which platinum treatment response data are available were collected from the TCGA database (https://portal.gdc.cancer.gov/). For breast cancer cell lines, WES and RNA-seq data were obtained from the CCLE database (https://portals.broadinstitute.org/ccle).

**Example 2. Calculation of genomic HRD (gHRD)**

The genomic scar values of the samples collected in Example 1 were calculated using existing software (https://github.com/GerkeLab/TCGAhrd) based on Affymetrix SNP data, and for mutational signature 3 based on WES data, values calculated using deconstructSigs in mSignatureDB (Po-Jung Huang. et al. mSignatureDB a database for deciphering mutational signatures in human cancers) in TCGA were used.

Here, gHRD(+) and gHRD(-) were distinguished from each other depending on the median values of genomic scar and mutational signature 3. In order to eliminate errors caused by inconsistency between indexes (median values of genomic scar and mutational signature 3), cases exceeding the median values of both indexes were classified as gHRD(+), and cases less than or equal to such median values were classified as gHRD(-).

**Example 3. Calculation of transcript usage (TU)**

TPM (transcripts per million) of a novel transcript was calculated through the stringtie 2-pass method using RNA-seq bam files as input in TCGA or CCLE. For the relevant process, reference was made to Mihaela Peratea et al. Nat. Protoc. Vol. 11(9), pp. 1650-1667, 2016.

For reference, the assembly step was performed using gencode v29 gtf (for CCLE, gencode v19 gtf was used), and using the merged gtf obtained by merging the gtf of individual samples therefrom as a new reference annotation, the second pass transcript quantification step was conducted.

For a de novo/novel transcript that cannot be annotated in the existing form, the gene of the most similar annotated transcript was assigned to the corresponding form using gffcompare for gene annotation.

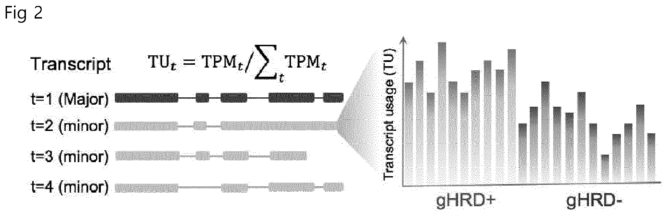

Thereby, TPM values of most transcripts expressed in individual samples were obtained, and based thereon, TU of each transcript was calculated using Equation 1 below (FIG. 2) .

$$\mathbf{TU}_t = \mathbf{TPM}_t \Big/ \sum_t \mathbf{TPM}_t$$

Equation 1:

Here, TPM represents transcripts per million.

**Example 4. Selection of minor isoform**

For isoform classification, the transcript annotation provided by the APPRIS database (Jose Manuel Rodriguez. et al. Nucleic Acids Res. Vol. 46(D1):D213-D217, 2018.) was used, and the version of the relevant annotation was 2019.

02. v29.

In order to define the minor isoform (alternative transcript), the criteria provided by APPRIS were used without change, and principals (1 to 5), which are transcripts encoding functionally or evolutionarily well-conserved proteins, were judged as major forms and all were removed. Thereby, a minor transcript set composed of alternative or not-report was selected.

In order to filter genes with low expression levels, count per million (CPM) normalization for each gene was performed through HTSeq read count data for each gene, only genes with mean CPM > 1 in the entire sample were selected, and only cases where the transcript usage (TU) was 3% or more (TU $\geq$ 0.03) among such genes were selected as the final TU set.

**Example 5. Discovery of gHRD-related aberrant TU (aTU) and analysis of function**

**5-1. Discovery of aTU**

As shown in FIG. 2, aTU was discovered through comparison between gHRD(+) and gHRD(-) groups. For more precise classification, samples with genomic scar values in the top 50% within gHRD(+) and samples with genomic scar values in the bottom 50% within gHRD(-) were additionally selected.

Statistical significance of a difference in TU between these two groups was determined using the Mann-Whitney U test, and correction was performed using Benjamini-Hochberg correction (BH correction).

Cases in which the TU of a specific minor isoform in gHRD(+) was significantly high (FDR < 10) were defined as "aTU in gHRD(+)", whereas cases in which the TU of a specific minor isoform in gHRD(-) was significantly high (FDR < 10) were defined as "aTU in gHRD(-)".

**5-2. Results of analysis of function**

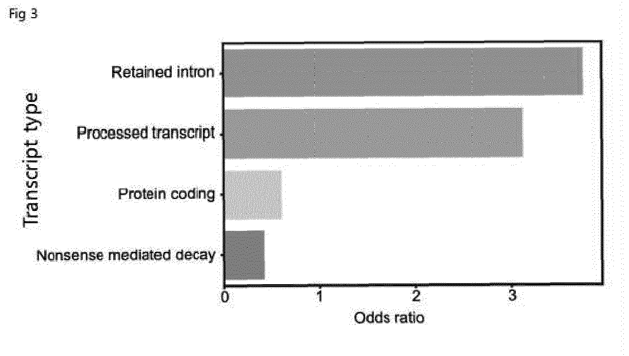

For breast cancer patient samples, three gene ontology parent terms including DNA repair, replication, and recombination were merged to define HR-related terms (1,033 genes). Based on results of GSEA (prerank) analysis using the same, as shown in FIG. 3, it was confirmed that DNA repair-related genes were statistically clustered.

The transcripts were sorted based on the U value of the Mann-Whitney U test (FDR < 50) in the method of Example 5-1, and the HR-related terms were overlapped in the relevant matrix. Here, the higher the U value, the more the minor isoform of the relevant gene is expressed in the gHRD(+) sample.

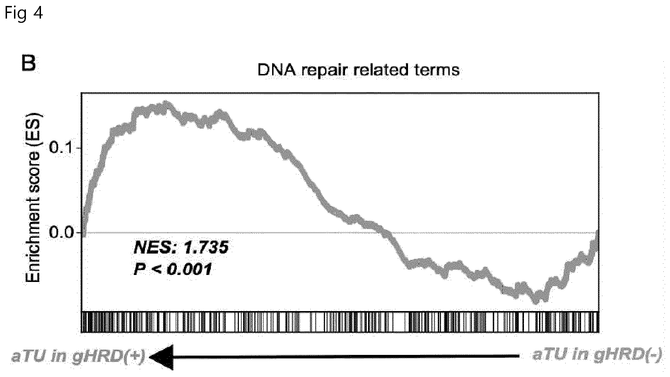

The method of Example 5-1 was also applied to ovarian cancer to discover aTU. For ovarian cancer, BH correction was not applied due to insufficient number of genes, and P < 0.05 of Mann-Whitney U test was applied to determine the most significant aTU for each gene. Subsequently, based on results of distinguishing aTU in gHRD(+) and aTU in gHRD(+) from each other depending on the orientation of each gene, as shown in FIG. 4, it was confirmed that DNA repair-related genes were statistically clustered.

**Example 6. Construction of transcriptional HRD (tHRD) model**

**6-1. Preparation of input data for model construction**

Based on results of selection of DNA repair-related genes among the genes obtained by the method of Example 5-1, 36 genes (104 minor transcripts) in breast cancer and 25 genes (89 minor transcripts) in ovarian cancer were selected.

[Table 1]

| List of genes and transcripts selected for breast cancer | | |
|---|---|---|
| **Genes** | **Minor isoforms** | **Isoforms type** |
| RPAIN | ENST00000327154.10-RPAIN | protein_coding |
| | ENST00000381208.9-RPAIN | protein_coding |
| | ENST00000405578.8-RPAIN | protein_coding |
| | ENST00000536255.6-RPAIN | protein_coding |
| | ENST00000572174.5-RPAIN | retained_intron |
| | ENST00000573126.1-RPAIN | retained_intron |
| | ENST00000575112.5-RPAIN | nonsense_mediated_decay |
| MUS81 | ENST00000524647.5-MUS81 | nonsense_mediated_decay |
| | ENST00000525006.1-MUS81 | processed_transcript |
| | ENST00000525224.5-MUS81 | processed_transcript |
| | ENST00000525768.5-MUS81 | protein_coding |
| | ENST00000529374.5-MUS81 | protein_coding |
| | ENST00000530282.1-MUS81 | retained_intron |
| FEN1 | ENST00000535307.1-FEN1 | protein_coding |
| | ENST00000535723.1-FEN1 | protein_coding |
| SMARCB1 | ENST00000344921.11-SMARCB1 | protein_coding |
| | ENST00000407422.8-SMARCB1 | protein_coding |
| DCLRE1C | ENST00000378242.1-DCLRE1C | protein_coding |
| | ENST00000489845.1-DCLRE1C | processed_transcript |
| CDCA5 | ENST00000404147.3-CDCA5 | protein_coding |
| | ENST00000479032.6-CDCA5 | retained_intron |
| RAD51AP1 | ENST00000228843.13-RAD51AP1 | protein_coding |
| | ENST00000442992.6-RAD51AP1 | nonsense_mediated_decay |
| | ENST00000544029.1-RAD51AP1 | retained_intron |
| ALKBH2 | ENST00000536358.1-ALKBH2 | protein_coding |
| BABAM2 | ENST00000361704.6-BABAM2 | protein_coding |
| | ENST00000379632.6-BABAM2 | protein_coding |
| ATXN3 | ENST00000340660.10-ATXN3 | protein_coding |
| | ENST00000393287.9-ATXN3 | protein_coding |
| | ENST00000503767.5-ATXN3 | protein_coding |
| BRIP1 | ENST00000577598.5-BRIP1 | protein_coding |
| KAT 5 | ENST00000525600.1-KAT5 | retained_intron |
| | ENST00000530446.5-KAT5 | protein_coding |
| | ENST00000533441.1-KAT5 | retained_intron |
| EXO1 | ENST00000423131.5-EXO1 | protein_coding |
| | ENST00000518483.5-EXO1 | protein_coding |
| | ENST00000521202.2-EXO1 | protein_coding |

(continued)

| List of genes and transcripts selected for breast cancer | | |
|---|---|---|
| **Genes** | **Minor isoforms** | **Isoforms type** |
| POLD1 | ENST00000593407.5-POLD1 | protein_coding |
| | ENST00000595904.6-POLD1 | protein_coding |
| | ENST00000596221.1-POLD1 | retained_intron |
| | ENST00000596648.1-POLD1 | retained_intron |
| | ENST00000597963.5-POLD1 | retained_intron |
| | ENST00000600859.5-POLD1 | nonsense_mediated_decay |
| CHEK1 | ENST00000427383.6-CHEK1 | protein_coding |
| | ENST00000498122.4-CHEK1 | nonsense_mediated_decay |
| | ENST00000532449.5-CHEK1 | protein_coding |
| | ENST00000544373.5-CHEK1 | protein_coding |
| RAD54L | ENST00000459678.2-RAD54L | nonsense_mediated_decay |
| | ENST00000472889.2-RAD54L | protein_coding |
| FANCI | ENST00000310775.11-FANCI | protein_coding |
| | ENST00000566895.5-FANCI | retained_intron |
| USP47 | ENST00000305481.10-USP47 | processed_transcript |
| | ENST00000529813.1-USP47 | retained_intron |
| MGME1 | ENST00000377704.4-MGME1 | protein_coding |
| | ENST00000377709.1-MGME1 | protein_coding |
| | ENST00000467391.1-MGME1 | processed_transcript |
| PARP3 | ENST00000398755.7-PARP3 | protein_coding |
| | ENST00000470601.5-PARP3 | retained_intron |
| | ENST00000475782.1-PARP3 | retained_intron |
| | ENST00000498510.1-PARP3 | protein_coding |
| RFC4 | ENST00000417876.1-RFC4 | protein_coding |
| | ENST00000418288.5-RFC4 | protein_coding |
| | ENST00000494047.5-RFC4 | retained_intron |
| FANCC | ENST00000490972.7-FANCC | protein_coding |
| MND1 | ENST00000504860.2-MND1 | protein_coding |
| | ENST00000509752.5-MND1 | nonsense_mediated_decay |
| FANCB | ENST00000452869.1-FANCB | protein_coding |
| | ENST00000489126.1-FANCB | retained_intron |
| PARP9 | ENST00000462315.5-PARP9 | protein_coding |
| | ENST00000471785.5-PARP9 | protein_coding |
| | ENST00000489652.1-PARP9 | retained_intron |
| SMC1A | ENST00000375340.10-SMC1A | protein_coding |
| | ENST00000463684.1-SMC1A | nonsense_mediated_decay |
| | ENST00000470241.2-SMC1A | protein_coding |

(continued)

| List of genes and transcripts selected for breast cancer | | |
|---|---|---|
| **Genes** | **Minor isoforms** | **Isoforms type** |
| DDB2 | ENST00000378601.7-DDB2 | nonsense_mediated_decay |
| TICRR | ENST00000560985.5-TICRR | protein_coding |
| | ENST00000561095.1-TICRR | nonsense_mediated_decay |
| RAD51 | ENST00000525066.5-RAD51 | nonsense_mediated_decay |
| | ENST00000527860.5-RAD51 | protein_coding |
| | ENST00000531277.2-RAD51 | nonsense_mediated_decay |
| UBE2T | ENST00000487227.6-UBE2T | retained_intron |
| ERCC1 | ENST00000013807.9-ERCC1 | protein_coding |
| | ENST00000340192.11-ERCC1 | protein_coding |
| | ENST00000423698.6-ERCC1 | protein_coding |
| | ENST00000592083.5-ERCC1 | protein_coding |
| | ENST00000592444.5-ERCC1 | protein_coding |
| | ENST00000592905.5-ERCC1 | retained_intron |
| MSHS | ENST00000375703.7-MSH5 | protein_coding |
| | ENST00000395853.5-MSH5 | protein_coding |
| | ENST00000463094.5-MSH5 | retained_intron |
| | ENST00000463144.5-MSH5 | nonsense_mediated_decay |
| | ENST00000467319.1-MSHS | retained_intron |
| | ENST00000494458.1-MSH5 | retained_intron |
| | ENST00000494646.1-MSHS | retained_intron |
| | ENST00000497269.5-MSH5 | nonsense_mediated_decay |
| UBE2V2 | ENST00000518360.5-UBE2V2 | nonsense_mediated_decay |
| | ENST00000521628.1-UBE2V2 | retained_intron |
| | ENST00000523432.5-UBE2V2 | protein_coding |
| TRIP13 | ENST00000508456.1-TRIP13 | processed_transcript |
| | ENST00000513435.1-TRIP13 | protein_coding |
| NEIL1 | ENST00000561643.5-NEIL1 | retained_intron |
| | ENST00000565121.1-NEIL1 | retained_intron |
| | ENST00000567393.5-NEIL1 | retained_intron |
| | ENST00000567547.1-NEIL1 | retained_intron |

[Table 2]

| List of genes and transcripts selected for ovarian cancer | | |
|---|---|---|
| **Genes** | **Minor isoforms** | **Isoform type** |
| VCP | ENST00000493886.5-VCP | retained_intron |

(continued)

| List of genes and transcripts selected for ovarian cancer | | |
|---|---|---|
| **Genes** | **Minor isoforms** | **Isoform type** |
| BLM | ENST00000560559.1-BLM | retained_intron |
| | ENST00000558825.5-BLM | retained_intron |
| | ENST00000560821.1-BLM | processed_transcript |
| | ENST00000560509.5-BLM | protein_coding |
| ABRAXAS1 | ENST00000515303.2-ABRAXAS1 | protein_coding |
| | ENST00000475656.6-ABRAXAS1 | nonsense_mediated_decay |
| | ENST00000504777.1-ABRAXAS1 | retained_intron |
| XPC | ENST00000476581.6-XPC | nonsense_mediated_decay |
| | ENST00000427795.2-XPC | retained_intron |
| RECQL4 | ENST00000534626.6-RECQL4 | protein_coding |
| RAD51AP1 | ENST00000544029.1-RAD51AP1 | retained_intron |
| | ENST00000544927.5-RAD51AP1 | protein_coding |
| | ENST00000228843.13-RAD51AP1 | protein_coding |
| | ENST00000442992.6-RAD51AP1 | nonsense_mediated_decay |
| UBB | ENST00000535788.1-UBB | protein_coding |
| | ENST00000578649.1-UBB | processed_transcript |
| SUMO1 | ENST00000409181.1-SUMO1 | protein_coding |
| | ENST00000409368.5-SUMO1 | protein_coding |
| EXO1 | ENST00000518483.5-EXO1 | protein_coding |
| CHEK2 | ENST00000472807.1-CHEK2 | retained_intron |
| | ENST00000403642.5-CHEK2 | protein_coding |
| | ENST00000382580.6-CHEK2 | protein_coding |
| | ENST00000402731.5-CHEK2 | protein_coding |
| | ENST00000433728.5-CHEK2 | nonsense_mediated_decay |
| UIMC1 | ENST00000510376.1-UIMC1 | retained_intron |
| | ENST00000510698.2-UIMC1 | protein_coding |
| | ENST00000503273.1-UIMC1 | processed_transcript |
| | ENST00000505229.1-UIMC1 | retained_intron |
| RPS3 | ENST00000527273.5-RPS3 | protein_coding |
| | ENST00000526608.5-RPS3 | protein_coding |
| | ENST00000534440.5-RPS3 | protein_coding |
| | ENST00000532872.5-RPS3 | nonsense_mediated_decay |
| TNKS1BP1 | ENST00000527207.1-TNKS1BP1 | protein_coding |
| | ENST00000427750.2-TNKS1BP1 | retained_intron |
| | ENST00000532273.5-TNKS1BP1 | retained_intron |
| MUTYH | ENST00000531105.5-MUTYH | protein_coding |

(continued)

| List of genes and transcripts selected for ovarian cancer | | |
|---|---|---|
| **Genes** | **Minor isoforms** | **Isoform type** |
| | ENST00000355498.6-MUTYH | protein_coding |
| | ENST00000478796.5-MUTYH | retained_intron |
| | ENST00000533178.5-MUTYH | nonsense_mediated_decay |
| | ENST00000482094.5-MUTYH | retained_intron |
| | ENST00000528013.6-MUTYH | protein_coding |
| | ENST00000466231.1-MUTYH | retained_intron |
| RMI2 | ENST00000576027.1-RMI2 | protein_coding |
| | ENST00000572992.1-RMI2 | processed_transcript |
| GADD45A | ENST00000370985.4-GADD45A | protein_coding |
| | ENST00000484245.1-GADD45A | retained_intron |
| PARP2 | ENST00000429687.7-PARP2 | protein_coding |
| | ENST00000530598.2-PARP2 | retained_intron |
| | ENST00000527915.5-PARP2 | protein_coding |
| | ENST00000527384.1-PARP2 | retained_intron |
| | ENST00000532299.5-PARP2 | retained_intron |
| ASCC1 | ENST00000486689.6-ASCC1 | protein_coding |
| | ENST00000534259.1-ASCC1 | retained_intron |
| USP45 | ENST00000513344.1-USP45 | retained_intron |
| | ENST00000508908.1-USP45 | protein_coding |
| | ENST00000329966.10-USP45 | protein_coding |
| | ENST00000496090.6-USP45 | protein_coding |
| UBE2A | ENST00000371569.6-UBE2A | retained_intron |
| | ENST00000469205.2-UBE2A | retained_intron |
| | ENST00000346330.6-UBE2A | protein_coding |
| RAD51B | ENST00000460526.5-RAD51B | processed_transcript |
| | ENST00000390683.7-RAD51B | protein_coding |
| | ENST00000487861.5-RAD51B | protein_coding |
| | ENST00000554183.1-RAD51B | processed_transcript |
| NSMCE4A | ENST00000489266.5-NSMCE4A | processed_transcript |
| | ENST00000369017.5-NSMCE4A | protein_coding |
| | ENST00000459911.5-NSMCE4A | processed_transcript |
| | ENST00000483541.1-NSMCE4A | retained_intron |
| | ENST00000468209.5-NSMCE4A | processed_transcript |
| POLE2 | ENST00000554396.5-POLE2 | protein_coding |

(continued)

| List of genes and transcripts selected for ovarian cancer | | |
| --- | --- | --- |
| **Genes** | **Minor isoforms** | **Isoform type** |
| | ENST00000553805.2-POLE2 | protein_coding |
| | ENST00000556937.5-POLE2 | processed_transcript |
| | ENST00000539565.6-POLE2 | protein_coding |
| | ENST00000554851.5-POLE2 | retained_intron |
| | ENST00000554377.1-POLE2 | retained_intron |
| | ENST00000555724.5-POLE2 | processed_transcript |
| | ENST00000554671.5-POLE2 | processed_transcript |
| ERCC1 | ENST00000592083.5-ERCC1 | protein_coding |
| | ENST00000423698.6-ERCC1 | protein_coding |
| | ENST00000013807.9-ERCC1 | protein_coding |
| | ENST00000592444.5-ERCC1 | protein_coding |
| | ENST00000591636.5-ERCC1 | protein_coding |
| | ENST00000340192.11-ERCC1 | protein_coding |
| | ENST00000592905.5-ERCC1 | retained_intron |
| RHNO1 | ENST00000366285.5-RHNO1 | protein_coding |
| | ENST00000464682.2-RHNO1 | processed_transcript |
| | ENST00000535978.5-RHNO1 | nonsense_mediated_decay |
| | ENST00000461997.5-RHNO1 | protein_coding |

For breast cancer, 104 transcripts available in the CCLE data set used for the drug response test among 115 transcripts were prepared as input for a final model, and for ovarian cancer, all transcripts were prepared as input.

**6-2. Model construction**

Random forest was used as a tHRD prediction model.

For both breast cancer and ovarian cancer, positive and negative prediction models were constructed based on gHRD, and for ovarian cancer, responsive and resistant prediction models were additionally created based on platinum response.

The ratio of the training set and the test set was split at 7:3, hyperparameter tuning was performed 100 times using the RandomizedSearchCV package, and the optimal hyperparameter was determined by measuring the mean validation accuracy through 3-fold cross validation for each tuning.

A prediction model was constructed by learning the model with the hyperparameter obtained through RandomizedSearchCV using the RandomForestClassifier of the sklearn.ensemble module.

**Example 7. Analysis of drug response**

**7-1. Breast cancer**

Using the somatic mutation data of CCLE cell lines as input, 96 class types were classified through Rpackage deconstrucSigs, Sgenome.Hsapiens.UCSC.hg19 was set as a reference, and each signature value was calculated using non-negative matrix factorization (NMF) for pre-defined COSMIC signature.

Here, in order to correct the erroneous signature assignment resulting from the fitting method, signature 3 values fitted with the COSMIC signature set specific to breast cancer were calculated with reference to Francesco Maura. et al. Nat. Communications, Vol. 10:2069, 2019. Based thereon, the cell line belonging to the top 250 of signature 3 values was defined as gHRD(+) and the rest as gHRD(-) .

For comparison, also, signature 3 fitted for the entire COSMIC signature, which is not specific to breast cancer, was calculated.

Through 104 TU data for 36 breast cancer cell lines for which Genomics of Drug Sensitivity in Cancer (GDSC) data is available, a difference in drug response between tHRD(+) and tHRD(-) predicted from the breast cancer random forest model was measured, and for drug susceptibility, IC50 values for PARP inhibitors (olaparib, rucaparib, veliparib, talazoparib) and other DNA damaging agents (bleomycin, cisplatin, doxorubicin, etoposide, SN-38) from 2020. 02 updated data provided by GDSC were used. Comparison of IC50 values between the two groups was performed using the Mann-Whitney U test.

For precision and recall, the number of true positives, false positives, true negatives, and false negatives was measured based on the median value of IC50 while changing the signature 3 value and the tHRD prediction value.

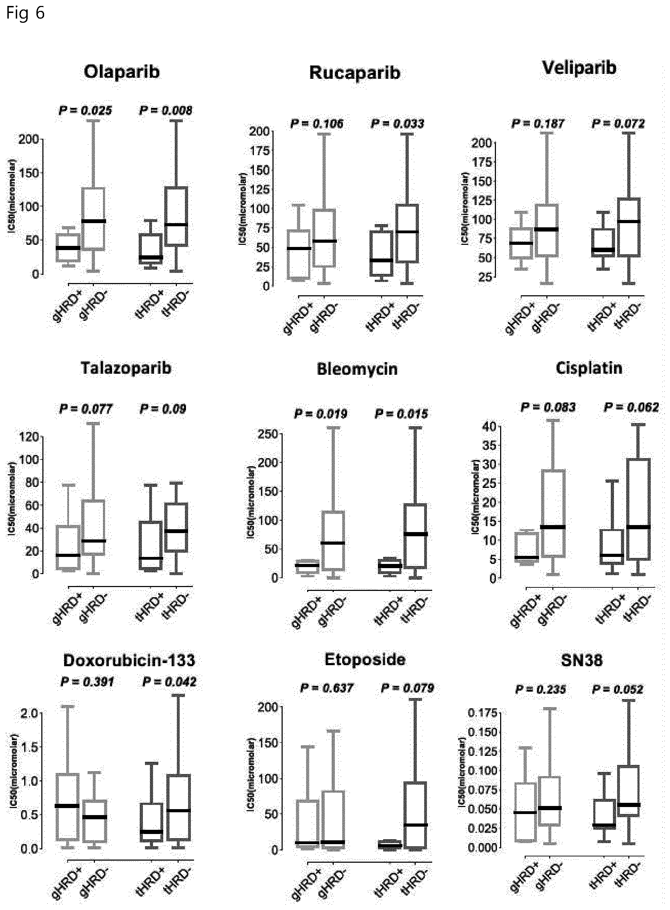

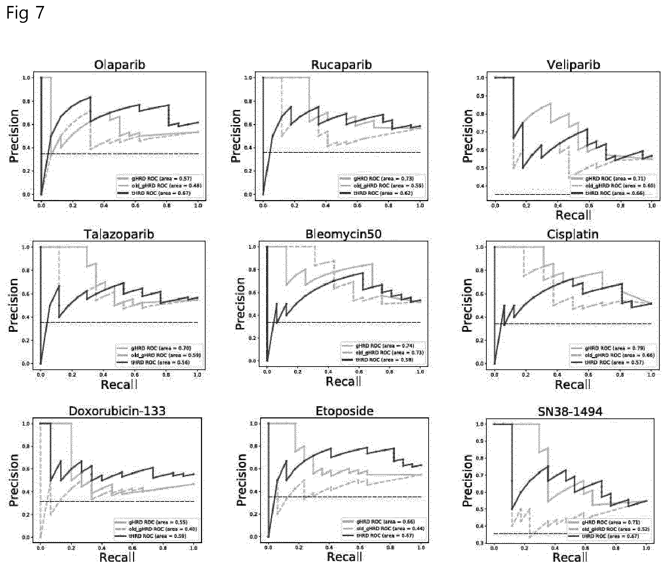

Thereby, it was confirmed that the accuracy of the tHRD method was vastly superior to that of the gHRD method, as shown in FIGs. 6 and 7.

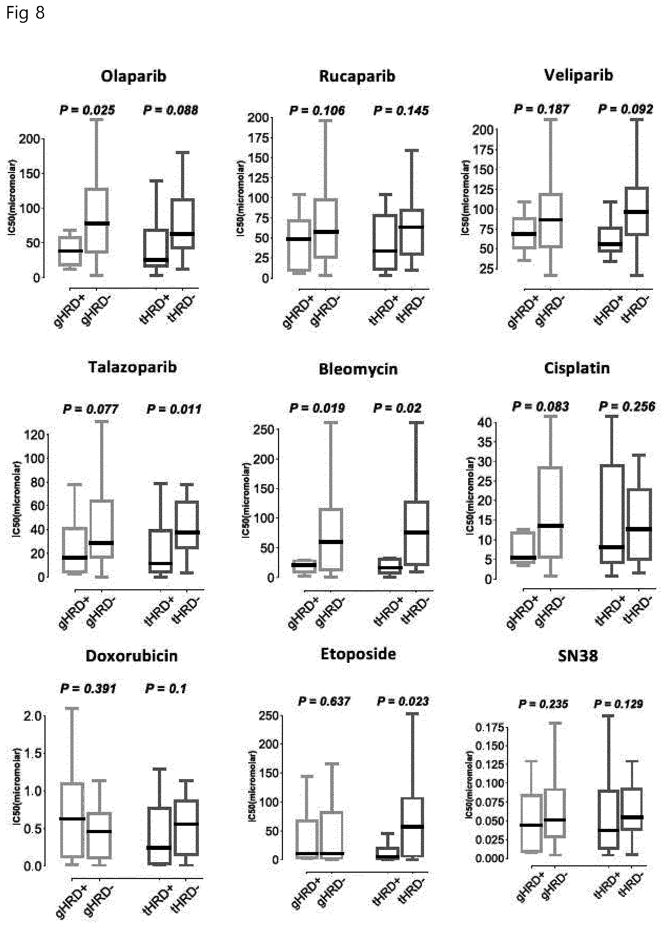

In addition, based on results of predicting drug response of breast cancer cell lines through the above method using only 20 major transcripts among 104 transcripts, as shown in Table 3 below, it was confirmed that the performance was still superior compared to the gHRD method, as shown in FIG. 8.

[Table 3]

| List of 20 major transcripts for breast cancer | |
|---|---|
| **Gene** | **Minor Isoform** |
| RPAIN | ENST00000536255.6-RPAIN |
| MUS81 | ENST00000525006.1-MUS81 |
| DCLRE1C | ENST00000489845.1-DCLRE1C |
| CDCAS | ENST00000404147.3-CDCA5 |
| ATXN3 | ENST00000393287.9-ATXN3 |
| CHEK1 | ENST00000498122.4-CHEK1 |
| CHEK1 | ENST00000544373.5-CHEK1 |
| PARP3 | ENST00000398755.7-PARP3 |
| RFC4 | ENST00000417876.1-RFC4 |
| MND1 | ENST00000509752.5-MND1 |
| FANCB | ENST00000452869.1-FANCB |
| PARP9 | ENST00000462315.5-PARP9 |
| SMC1A | ENST00000463684.1-SMC1A |
| TICRR | ENST00000560985.5-TICRR |
| RAD51 | ENST00000525066.5-RAD51 |
| UBE2T | ENST00000487227.6-UBE2T |
| ERCC1 | ENST00000013807.9-ERCC1 |
| ERCC1 | ENST00000592444.5-ERCC1 |
| NEIL1 | ENST00000565121.1-NEIL1 |
| NEIL1 | ENST00000567547.1-NEIL1 |

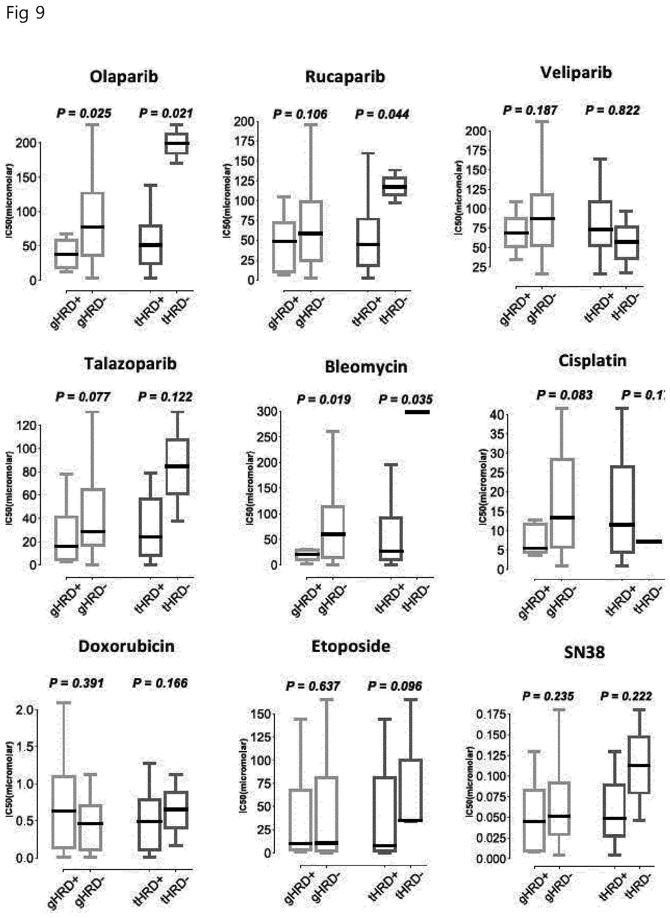

In addition, based on results of predicting drug response of breast cancer cell lines through the above method using only 10 major transcripts among the 104 transcripts, as shown in Table 4 below, it was confirmed that the performance was still superior compared to the gHRD method, as shown in FIG. 9.

[Table 4]

| List of 10 major transcripts for breast cancer | |
|---|---|
| Gene | Minor isoform |
| DCLRE1C | ENST00000489845.1-DCLRE1C |
| CDCAS | ENST00000404147.3-CDCA5 |
| RFC4 | ENST00000417876.1-RFC4 |
| FANCB | ENST00000452869.1-FANCB |
| SMC1A | ENST00000463684.1-SMC1A |
| TICRR | ENST00000560985.5-TICRR |
| RAD51 | ENST00000525066.5-RAD51 |
| ERCC1 | ENST00000013807.9-ERCC1 |
| NEIL1 | ENST00000565121.1-NEIL1 |
| NEIL1 | ENST00000567547.1-NEIL1 |

**7-2. Ovarian cancer**

In order to classify platinum response of TCGA ovarian cancer patients using the method of Example 7-1, among patients who received first-line therapy suggested in literature (Victor M. Villalobos. et al. JCO Clinical Cancer Informatics, Vol.2, pp.1-16, 2018), patients with progression within 6 months were classified as platinum resistant, and patients without progression were classified as platinum responsive. Among a total of 450 patients provided in the literature, 162 patients for whom RNA-seq, genomic scar, and signature 3 were all available were used for an ovarian cancer prediction model.

As shown in FIG. 10, it was confirmed that the accuracy of the tHRD-based method was superior to that of the gHRD method, and the patient survival rate when classified by tHRD also showed a clear difference compared to when classified by gHRD.

Also, based on results of predicting drug response of ovarian cancer patients through the above method using only 10 major transcripts among 89 transcripts, as shown in Table 5 below, it was confirmed that the performance was comparable to or still superior to that of the gHRD method, as shown in FIG. 11.

[Table 5]

| List of 10 major transcripts for ovarian cancer | |
|---|---|
| Gene | Minor isoform |
| SUMO1 | ENST00000409368.5-SUMO1 |
| CHEK2 | ENST00000403642.5-CHEK2 |
| MUTYH | ENST00000528013.6-MUTYH |
| RMI2 | ENST00000576027.1-RMI2 |
| PARP2 | ENST00000527915.5-PARP2 |
| UBE2A | ENST00000346330.6-UBE2A |
| RAD51B | ENST00000487861.5-RAD51B |
| NSMCE4A | ENST00000468209.5-NSMCE4A |
| POLE2 | ENST00000556937.5-POLE2 |
| ERCC1 | ENST00000591636.5-ERCC1 |

Furthermore, instead of learning the prediction model based on gHRD as in Example 7-1, additional prediction models were constructed based on platinum response depending on the presence or absence of progression within 6 months as suggested in the above literature and performance thereof was measured.

Thereby, as shown in FIG. 12, it was confirmed that the prediction performance of the tHRD-based method was superior to that of the gHRD method, and the patient survival rate when classified by tHRD also showed a clear difference from when classified by gHRD.

### 7-3. Verification of model performance in actual ovarian cancer patients

In order to verify the optimal reference point (0.4841) obtained by applying the model constructed in Example 6 (an artificial intelligence model trained to determine the presence or absence of genomic HRD using the TU values of DNA repair-related gene isoforms of the TCGA-OV sample as input data of a random forest model) to the RNA-seq data of platinum chemotherapy-treated ovarian cancer patients (n=27), HRD classes were sorted by predicting response with MGI DNB platform-based RNA sequencing data of independent platinum chemotherapy-treated ovarian cancer samples (n=20).

Thereby, based on cancer metastasis within 6 months after drug treatment, the model prediction result showed high specificity (1.0), and based on cancer metastasis within 12 months, the model prediction result showed specificity (0.78) and sensitivity (0.63) (Table 6).

[Table 6]

Results of tHRD classification based on cancer metastasis period within 6 months or 12 months and optimal reference point

| sample ID | Platinum response (6 months) | 12 months | PFS | tHRD | binary tHRD |
|---|---|---|---|---|---|
| OV-F047 | Sensitive | Sensitive | 22 | 0.586562387 | Positive |
| OV-F024 | Sensitive | Sensitive | 15 | 0.539223472 | Positive |
| OV-F028 | Sensitive | Sensitive | 13 | 0.528846263 | Positive |
| OV-F066 | Sensitive | Sensitive | 12 | 0.51019048 | Positive |
| OV-F073 | Sensitive | Resistant | 11 | 0.508377636 | Positive |
| OV-F044 | Sensitive | Sensitive | 27 | 0.505550919 | Positive |
| OV-F096 | Sensitive | Resistant | 11 | 0.496737301 | Positive |
| OV-F014 | Sensitive | Sensitive | 37 | 0.496412016 | Positive |
| OV-F003 | Sensitive | Sensitive | 34 | 0.490063076 | Positive |
| OV-F023 | Sensitive | Sensitive | 36 | 0.465662961 | Negative |
| OV-F094 | Sensitive | Resistant | 10 | 0.448342136 | Negative |
| OV-F071 | Sensitive | Resistant | 8 | 0.444047927 | Negative |
| OV-F018 | Sensitive | Sensitive | 27 | 0.438797679 | Negative |
| OV-F048 | Sensitive | Sensitive | 30 | 0.434881661 | Negative |
| OV-F095 | Resistant | Resistant | 5 | 0.412909566 | Negative |
| OV-F032 | Sensitive | Resistant | 27 | 0.406515814 | Negative |
| OV-F038 | Resistant | Resistant | 5 | 0.397129191 | Negative |
| OV-F039 | Sensitive | Resistant | 10 | 0.390879399 | Negative |
| OV-F049 | Sensitive | Resistant | 6 | 0.384056543 | Negative |
| OV-F006 | Resistant | Resistant | 4 | 0.35179428 | Negative |

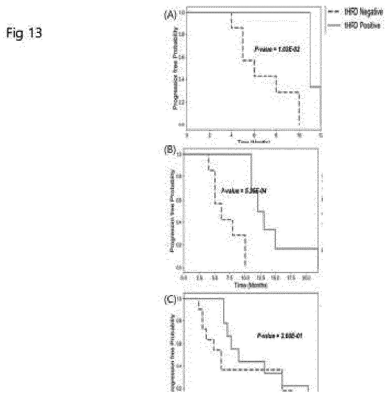

In addition, based on results of survival analysis through cancer metastasis period (progression-free survival (PFS)), it was confirmed that patients classified as HRD positive by the previously obtained optimal reference value (0.4841) had a statistically significantly low incidence of cancer metastasis in less than 2 years (FIG. 13).

Having described specific parts of the present invention in detail above, it will be obvious to those skilled in the art that these specific descriptions are only preferred embodiments, and the scope of the present invention is not limited thereby. Accordingly, the substantial scope of the present invention will be defined by the appended claims and equivalents thereto.

[Industrial Applicability]

According to the present invention, a method of determining susceptibility to a PARP inhibitor or DNA damaging agent is capable of determining susceptibility in real time with high accuracy using information of transcripts transcribed from genes and is thus useful, unlike existing methods of determining susceptibility to PARP inhibitors or DNA damaging agents based on genetic mutation information.

**Claims**

1. A method of determining susceptibility to a PARP inhibitor or DNA damaging agent, comprising:

   a) extracting a nucleic acid from a biological sample and then obtaining an expression level of each of non-functional transcripts of DNA repair-related genes;
   b) calculating transcript usage (TU) of non-functional transcripts for each gene based on the obtained expression level; and
   c) determining that there is susceptibility to a PARP (poly ADP-ribose polymerase) inhibitor or DNA damaging agent (genotoxic drug) when a value obtained by analyzing the calculated TU is greater than or equal to a reference value.

2. The method according to claim 1, wherein the nucleic acid is RNA.

3. The method according to claim 1, wherein step a) is performed through a method comprising:

   a-i) collecting nucleic acid from blood, semen, vaginal cells, hair, saliva, urine, oral cells, cancer tissue cells, FFPE samples, and mixtures thereof;
   a-ii) obtaining purified nucleic acid by removing proteins, fats, and other residues from the collected nucleic acid using a salting-out method, a column chromatography method, or a bead method;
   a-iii) constructing a library by enriching DNA repair-related genes for the purified nucleic acid;
   a-iv) reacting the constructed library in a next-generation sequencer; and
   a-v) obtaining nucleic acid sequence information (reads) from the next-generation sequencer.

4. The method according to claim 1, wherein the DNA repair-related genes are at least 10 genes selected from the group consisting of ABL1, ALKBH1, APEX1, APTX, ASF1A, ATM, ATP23, ATR, ATRX, ATXN3, BLM, BRCA1, BRCA2, BTG2, CCNO, CDKN2D, CEBPG, CIB1, CSNK1D, CSNK1E, DDB1, DDB2, ERCC1, ERCC2, ERCC3, ERCC4, ERCC5, ERCC6, ERCC8, EXO1, FANCA, FANCC, FANCG, FEN1, GADD45A, GADD45G, GTF2H1, GTF2H4, HMGB1, HMGB1P10, HMGB2, HUS1, IGHMBP2, KAT5, LIG1, LIG3, LIG4, MLH1, MMS19, MNAT1, MPG, MRE11, MSH2, MSH3, MSH5, MSH6, MUTYH, NBN, NHEJ1, NTHL1, OGG1, PARP1, PARP3, PMS1, PMS2, PMS2P1, PNKP, POLA1, POLD1, POLE, POLE2, POLG, POLH, POLI, POLL, POLQ, PRKCG, RAD1, RAD17, RAD21, RAD23A, RAD23B, RAD50, RAD51, RAD51B, RAD51C, RAD52, RAD54B, RAD54L, RAD9A, RBBP8, RECQL, RECQL4, RECQL5, REV1, RFC3, RPA1, RPAIN, RUVBL2, SETX, SMC1A, SMUG1, SOD1, SUMO1, TDG, TNP1, TP53, TP73, TREX2, UBE2A, UBE2B, UBE2N, UBE2V1, UBE2V2, UNG, UPF1, UVRAG, VCP, WRNIP1, XAB2, XPC, XRCC2, XRCC3, XRCC4, XRCC6, BABAM2, BRIP1, CDCA5, CHEK1, DCLRE1C, FANCB, FANCI, MGME1, MND1, MUS81, NEIL1, PARP9, RAD51AP1, RFC4, SMARCB1, TICRR, TRIP13, UBE2T, USP47, ABRAXAS1, ASCC1, CHEK2, NSMCE4A, PARP2, RAD51AP1, RHNO1, RMI2, RPS3, TNKS1BP1, UBB, UIMC1, and USP45.

5. The method according to claim 4, wherein, when the PARP inhibitor or DNA damaging agent is applied to breast cancer, the DNA repair-related genes are at least 10 genes selected from the group consisting of ALKBH2, ATXN3, BABAM2, BRIP1, CDCA5, CHEK1, DCLRE1C, DDB2, ERCC1, EXO1, FANCB, FANCC, FANCI, FEN1, KAT5, MGME1, MND1, MSH5, MUS81, NEIL1, PARP3, PARP9, POLD1, RAD51, RAD51AP1, RAD54L, RFC4, RPAIN, SMARCB1, SMC1A, TICRR, TRIP13, UBE2T, UBE2V2, and USP47.

6. The method according to claim 4, wherein, when the PARP inhibitor or DNA damaging agent is applied to ovarian cancer, the DNA repair-related genes are at least 10 genes selected from the group consisting of ABRAXAS1, ASCC1, BLM, CHEK2, ERCC1, EXO1, GADD45A, MUTYH, NSMCE4A, PARP2, POLE2, RAD51AP1, RAD51B, RECQL4, RHNO1, RMI2, RPS3, SUMO1, TNKS1BP1, UBB, UBE2A, UIMC1, USP45, VCP, and XPC.

7. The method according to claim 1, wherein the non-functional transcripts in step a) are minor isoforms.

8. The method according to claim 1, wherein calculating the transcript usage (TU) of the non-functional transcripts for each gene in step b) is performed using Equation 1 below:

Equation 1

$$\mathrm{TU}_t = \mathrm{TPM}_t / \sum_t \mathrm{TPM}_t$$

in which TPM represents transcripts per million.

9. The method according to claim 1, wherein obtaining the value by analyzing the calculated TU in step c) is performed in a manner in which TU values of non-functional transcripts overexpressed in a sample known to be genomic homologous recombination deficiency (gHRD) positive or drug responsive are multiplied by a specific weight and summed to obtain a determination value in a specific range, or which uses a decision-making process that makes a final decision according to an aspect in which a TU value of each non-functional transcript exceeds a specific reference value.

10. The method according to claim 9, wherein the manner in which the TU values of the non-functional transcripts overexpressed in the sample known to be gHRD positive or drug responsive are multiplied by the specific weight and summed to obtain the determination value in the specific range is performed by multiplying TU values of non-functional transcripts corresponding to the non-functional transcripts overexpressed in the sample known to be gHRD positive or drug responsive by a weight, followed by summing and then normalization to a value between 0 and 1.

11. The method according to claim 1, wherein the reference value is 0.5 to 1.

12. The method according to claim 9, wherein obtaining the value by analyzing the calculated TU is performed using an artificial intelligence model.

13. The method according to claim 12, wherein the artificial intelligence model enables construction of a prediction model through machine learning using combinations of weights assigned to TU values of non-functional transcripts for each gene overexpressed in a gHRD-positive or drug-responsive sample and a reference value for a determination value resulting from summing the weighted TU values so as to reflect transcript expression patterns of various patients, or enables construction of a prediction model through machine learning by structuring a decision-making process of comparing TU values of non-functional transcripts for each gene overexpressed in a gHRD-positive or drug-responsive sample with a reference value therefor so as to reflect transcript expression patterns of various patients.

14. The method according to claim 13, wherein the machine learning is performed through at least one process selected from the group consisting of K-nearest neighbors, linear regression, logistic regression, support vector machine (SVM), decision tree, random forest, and neural network.

15. The method according to claim 1, wherein the PARP inhibitor is selected from the group consisting of AZD2281 (olaparib), ABT888 (veliparib), AG014699 (rucaparib), MK-4827 (niraparib), BMN-673 (talazoparib), BSI201 (iniparib), BGP15 (O-(3-piperidino-2-hydroxy-1-propyl)nicotinic amidoxime), INO1001 (3-aminobenzamide), ONO2231, nicotinamide, 3-aminobenzamide, 3,4-dihydro-5-[4-(1-piperidinyl)butoxy]-1(2H)-isoquinolone, benzamide, quinolone, isoquinolone, benzopyrone, cyclic benzamide, benzimidazole, indole, and phenanthridinone.

16. The method according to claim 1, wherein the DNA damaging agent is selected from the group consisting of bleomycin, cisplatin, carboplatin, oxaliplatin, nedaplatin, doxorubicin, etoposide, and SN38.

17. An apparatus for determining susceptibility to a PARP inhibitor or DNA damaging agent for use in the method according to any one of claims 1 to 16, comprising:

(1) an information acquisition unit configured to extract a nucleic acid from a biological sample and obtain an expression level of each of non-functional transcripts of DNA repair-related genes;
(2) a calculation unit configured to calculate transcript usage (TU) of non-functional transcripts for each gene based on the obtained expression level; and

(3) a susceptibility determination unit configured to determine that there is susceptibility to a PARP (poly ADP-ribose polymerase) inhibitor or DNA damaging agent (genotoxic drug) when a value obtained by analyzing the calculated TU is greater than or equal to a reference value.

18. A computer-readable recording medium for use in the method according to any one of claims 1 to 16, wherein the medium comprises instructions configured to be executed by a processor that determines susceptibility to a PARP inhibitor or DNA damaging agent, comprising:

a) extracting a nucleic acid from a biological sample and then obtaining an expression level of each of non-functional transcripts of DNA repair-related genes;
b) calculating transcript usage (TU) of non-functional transcripts for each gene based on the obtained expression level; and
c) determining that there is susceptibility to a PARP (poly ADP-ribose polymerase) inhibitor or DNA damaging agent (genotoxic drug) when a value obtained by analyzing the calculated TU is greater than or equal to a reference value.

19. A targeted RNA sequencing (targeted RNA-Seq) kit for use in the method according to any one of claims 1 to 16, comprising:

a probe configured to capture a transcript of a DNA repair-related gene group; and
a primer configured to amplify the captured transcript.

Fig 1

```
┌─────────────────────────────────────────────────────────────┐
│   Obtain RNA fragment (reads) data from biological sample     │
│              by massive parallel sequencing                   │
└─────────────────────────────────────────────────────────────┘
                              │
                              ▼
┌─────────────────────────────────────────────────────────────┐
│       Align RNA fragment data to human reference genome       │
└─────────────────────────────────────────────────────────────┘
                              │
                              ▼
┌─────────────────────────────────────────────────────────────┐
│      Perform filtering depending on quality of aligned data   │
└─────────────────────────────────────────────────────────────┘
                              │
                              ▼
┌─────────────────────────────────────────────────────────────┐
│    Select non−functional transcripts of DNA repair−related    │
│            genes to be used for determination                 │
└─────────────────────────────────────────────────────────────┘
                              │
                              ▼
┌─────────────────────────────────────────────────────────────┐
│     Obtain expression levels of non−functional transcripts    │
│                      for each gene                            │
└─────────────────────────────────────────────────────────────┘
                              │
                              ▼
┌─────────────────────────────────────────────────────────────┐
│   Calculate transcript usage (TU) of non−functional transcripts│
│                      for each gene                            │
└─────────────────────────────────────────────────────────────┘
                              │
                              ▼
┌─────────────────────────────────────────────────────────────┐
│  Obtain output value by inputting TU to artificial intelligence│
│         model for drug susceptibility determination           │
└─────────────────────────────────────────────────────────────┘
                              │
                              ▼
┌─────────────────────────────────────────────────────────────┐
│   Determine drug susceptibility by comparing output value     │
│                    with reference value                       │
└─────────────────────────────────────────────────────────────┘
```

Fig 2

Transcript $\quad TU_t = TPM_t / \sum_t TPM_t$

t=1 (Major)

t=2 (minor)

t=3 (minor)

t=4 (minor)

Transcript usage (TU)

gHRD+ gHRD-

Fig 3

Fig 4

B DNA repair related terms

NES: 1.735
P < 0.001

aTU in gHRD(+) ← aTU in gHRD(-)

Fig 5

Fig 6

Fig 7

Fig 8

Fig 9

Fig 10

Fig 11

(A)

(B)

(C)

(D)

Fig 12

Fig 13

<div align="center">

**INTERNATIONAL SEARCH REPORT**

</div>

| | |
|---|---|
| | International application No. |
| | **PCT/KR2021/015800** |

| A. CLASSIFICATION OF SUBJECT MATTER |
|---|
| **G16B 15/30**(2019.01)i; **G16B 30/10**(2019.01)i; **G16B 20/20**(2019.01)i; **G16B 40/20**(2019.01)i; **C12Q 1/6869**(2018.01)i; **G06N 20/00**(2019.01)i; **G06N 3/08**(2006.01)i |
| According to International Patent Classification (IPC) or to both national classification and IPC |

| B. FIELDS SEARCHED |
|---|
| Minimum documentation searched (classification system followed by classification symbols) |
| G16B 15/30(2019.01); C12Q 1/68(2006.01) |
| Documentation searched other than minimum documentation to the extent that such documents are included in the fields searched |
| Korean utility models and applications for utility models: IPC as above <br> Japanese utility models and applications for utility models: IPC as above |
| Electronic data base consulted during the international search (name of data base and, where practicable, search terms used) |
| eKOMPASS (KIPO internal) & keywords: 상동성 재조합 결핍(Homologous Recombination Deficiency; HRD), PARP 저해제(poly ADP ribose polymerase inhibitor), 감수성(Susceptibility), 전사체 사용율(transcript usage), 유방암(breast cancer), 난소암(ovarian cancer) |

**C. DOCUMENTS CONSIDERED TO BE RELEVANT**

| Category* | Citation of document, with indication, where appropriate, of the relevant passages | Relevant to claim No. |
|---|---|---|
| X <br><br> Y | CHOI, Jung Kyoon. Identification of homologous recombination repair deficiency through the transcript usage analysis in breast cancer patients. 2019. 석사논문, 한국과학기술원, 바이오 및 뇌공학과 (Master's thesis, Department of Bio and Brain Engineering, Korea Advanced Institute of Science and Technology). pp. 1-42. <br>     See abstract; and pages 1, 5, 6, 10, 13, 16, 22, 27, 28, 33, 36, 37, 39 and 40. | 1,2,4-19 <br><br> 3 |
| Y | PERTEA, M. et al. StringTie enables improved reconstruction of a transcriptome from RNA-seq reads. Nature Biotechnology. 2015, vol. 33, no. 3, pp. 290-295, HHS Public Access Author manuscript version(pp. 1-20). <br>     See abstract; and page 10, third-fourth paragraphs. | 3 |
| A | US 2014-0364434 A1 (THE REGENTS OF THE UNIVERSITY OF CALIFONIA) 11 December 2014 (2014-12-11) <br>     See entire document. | 1-19 |

| ☑ Further documents are listed in the continuation of Box C. | ☑ See patent family annex. |
|---|---|

| | |
|---|---|
| \*   Special categories of cited documents: <br> "A"   document defining the general state of the art which is not considered to be of particular relevance <br> "D"   document cited by the applicant in the international application <br> "E"   earlier application or patent but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search | Date of mailing of the international search report |
|---|---|
| **10 February 2022** | **10 February 2022** |

| Name and mailing address of the ISA/KR | Authorized officer |
|---|---|
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
|---|
| **PCT/KR2021/015800** |

### C.     DOCUMENTS CONSIDERED TO BE RELEVANT

| Category* | Citation of document, with indication, where appropriate, of the relevant passages | Relevant to claim No. |
|---|---|---|
| A | KR 10-2016-0014565 A (THE ASAN FOUNDATION) 11 February 2016 (2016-02-11)<br>    See entire document. | 1-19 |
| A | KAWAZU, M. et al. Integrative analysis of genomic alterations in triple-negative breast cancer in association with homologous recombination deficiency. PLoS Genetics. 21 June 2017, vol. 13, no. 6, e100 6853(pp. 1-23).<br>    See entire document. | 1-19 |
| PX | KANG, H. G. et al. Aberrant transcript usage induces homologous recombination deficiency and predicts therapeutic responses. medRxiv. 02 September 2021, pp. 1-37.<br>    See abstract; pages 4-12 and 28-36; and figures 1-6. | 1-19 |

Form PCT/ISA/210 (second sheet) (July 2019)

**INTERNATIONAL SEARCH REPORT**
Information on patent family members

| | International application No. |
|---|---|
| | **PCT/KR2021/015800** |

| Patent document cited in search report | Publication date (day/month/year) | Patent family member(s) | Publication date (day/month/year) |
|---|---|---|---|
| US 2014-0364434 A1 | 11 December 2014 | WO 2013-133876 A1 | 12 September 2013 |
| KR 10-2016-0014565 A | 11 February 2016 | KR 10-1844541 B1 | 02 April 2018 |
| | | WO 2016-018089 A1 | 04 February 2016 |

Form PCT/ISA/210 (patent family annex) (July 2019)

## REFERENCES CITED IN THE DESCRIPTION

**Non-patent literature cited in the description**

- **GULHAN, D. C. et al.** *Nat. Genet.,* 2019, vol. 51, 912-919
- **ALEXANDROV, L. B. et al.** *Nature,* 2013, vol. 500, 415-421
- **ABKEVICH, V. et al.** *Br. J. Cancer.,* 2012, vol. 107, 1776-1782
- **POPOVA, T. et al.** *Cancer Res.,* 2012, vol. 72, 5454-5462
- **NICOLAI J. BIRKBAK.** *CANCER Discov.,* 2012, vol. 2, 367
- **NORQUIST, B. et al.** *J. Clin. Oncol.,* 2011, vol. 29, 3008-3015
- **CHAUDHURI, A. R. et al.** *Nature,* 2016, vol. 535, 382-387
- **MAURA, F. et al.** *Nat. Commun.,* 2019, vol. 10
- **WATKINS, J. A. et al.** *Breast Cancer Research,* 2014, vol. 16, 1-11
- **METZKER, M.** *Nature Biotechnology Reviews,* 2010, vol. 11, 31-46
- **MIHAELA PERATEA et al.** *Nat. Protoc.,* 2016, vol. 11 (9), 1650-1667
- **JOSE MANUEL RODRIGUEZ et al.** *Nucleic Acids Res.,* 2018, vol. 46 (D1), D213-D217
- **FRANCESCO MAURA et al.** *Nat. Communications,* 2019, vol. 10, 2069
- **VICTOR M. VILLALOBOS et al.** *JCO Clinical Cancer Informatics,* 2018, vol. 2, 1-16